(19) Europäisches Patentamt / European Patent Office / Office européen des brevets

(11) EP 2 170 253 B1

(12) **EUROPÄISCHE PATENTSCHRIFT**

(45) Veröffentlichungstag und Bekanntmachung des Hinweises auf die Patenterteilung:
**24.09.2014 Patentblatt 2014/39**

(21) Anmeldenummer: **08773820.9**

(22) Anmeldetag: **02.07.2008**

(51) Int Cl.:
***A61K 8/37*** $^{(2006.01)}$ ***A61K 8/42*** $^{(2006.01)}$
***A61Q 17/04*** $^{(2006.01)}$ ***A61K 8/41*** $^{(2006.01)}$
***A61K 8/49*** $^{(2006.01)}$ ***A61K 8/11*** $^{(2006.01)}$

(86) Internationale Anmeldenummer:
**PCT/EP2008/005408**

(87) Internationale Veröffentlichungsnummer:
**WO 2009/012871 (29.01.2009 Gazette 2009/05)**

(54) **UV-FILTER-KAPSEL**

UV- FILTER CAPSULES

CAPSULE FILTRANT LES UV

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **26.07.2007 DE 102007035567**

(43) Veröffentlichungstag der Anmeldung:
**07.04.2010 Patentblatt 2010/14**

(73) Patentinhaber: **BASF SE**
**67056 Ludwigshafen (DE)**

(72) Erfinder:
- **PFLUECKER, Frank**
  **64297 Darmstadt (DE)**
- **MUELLER, Bernd**
  **64673 Zwingenberg (DE)**
- **WITTE, Gabriele**
  **64572 Buettelborn (DE)**
- **ANDRE, Valerie**
  **67063 Ludwigshafen (DE)**

(56) Entgegenhaltungen:
**EP-A1- 0 717 982**
**EP-A1- 0 967 200**
**WO-A1-03/066209**
**WO-A2-00/72806**
**DE-A1-102006 006 413**
**US-A1- 2004 228 811**
**US-B1- 6 537 529**
**US-B1- 7 001 592**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 170 253 B1

Printed by Jouve, 75001 PARIS (FR)

## Beschreibung

Die vorliegende Erfindung betrifft UV-Filter-Kapseln, deren Verwendung zur Herstellung kosmetischer oder dermatologischer Formulierungen bzw. Dispersionen und kosmetische oder dermatologische Formulierungen, welche die Kapseln enthalten, sowie Verfahren zu deren Herstellung.

Es ist allgemein bekannt, dass der ultraviolette Teil der Sonnenstrahlung eine schädigende Wirkung auf die Haut hat. Während Strahlen mit einer Wellenlänge kleiner als 290 nm (sogenannter UVC-Bereich) von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Auch für die Strahlen im Bereich zwischen etwa 320 nm und 400 nm (UVA-Bereich) ist es erwiesen, dass sie zu einer Schädigung der elastischen und kollagenen Fasern des Bindegewebes führen, was die Haut vorzeitig altern lässt. Ferner sind diese Strahlen Ursache zahlreicher phototoxischer und photoallergischer Reaktionen. Der schädigende Einfluss der UVB-Strahlung kann durch die UVA-Strahlung noch verstärkt werden.

Die UV-Strahlung kann aber auch zu photochemischen Reaktionen führen, wobei dann die photochemischen Reaktionsprodukte in den Hautmetabolismus eingreifen. Ferner zählt UV-Strahlung zur ionisierenden Strahlung. Es besteht also das Risiko, dass auch ionische Spezien bei UV-Exposition entstehen, welche dann ihrerseits oxidativ in die biochemischen Prozesse einzugreifen vermögen.

Die heute üblichen Lichtschutzfilter in der Kosmetik und Dermatologie werden daher auch in UVA- bzw. UVB-Filter unterteilt. Zum Schutze gegen UVB-Strahlung sind zahlreiche Verbindungen bekannt, bei denen es sich zumeist um Derivate des 3-Benzylidencamphers (z.B. Eusolex® 6300), der 4-Aminobenzoesäure, der Zimtsäure, der Salicylsäure, des Benzophenons, des Triazins sowie auch des 2-Phenylbenzimidazols handelt. Zum Schutze gegen UVA-Strahlung werden häufig Dibenzoylmethan-Derivate eingesetzt, wie z.B. das 4-(tert.-Butyl)-4'-methoxy-dibenzoylmethan (Eusolex® 9020) oder das 4-Isopropyldibenzoylmethan (Eusolex® 8020), welche jedoch bei UV-Bestrahlung nicht unbegrenzt stabil sind.

In Anbetracht der oben genannten Ausführungen ist die Bereitstellung kosmetischer Produkte mit einem verbesserten, effektiveren oder optimierten Schutz der menschlichen Haut vor den schädigenden Wirkungen der UV-A und UV-B Strahlung von großer Bedeutung. Dabei ist es besonders wünschenswert Zubereitungen bereitzustellen, die bei möglichst geringer Einsatzmenge der einzelnen Komponenten eine gewünschte Wirkung erzielen. Dabei spielen die spezifische Extinktion der Lichtschutzmittel ebenso eine Rolle wie die Stabilität der mit ihnen hergestellten Emulsionen, deren toxikologische Unbedenklichkeit und deren Löslichkeit in den verwendeten Trägerstoffen (z.B. kosmetische Öle). Einige der bisher verwendeten Lichtschutzmittel zeichnen sich durch gute Extinktionseigenschaften aus, aber die geringe Löslichkeit dieser Substanzen verhindert deren optimalen Einsatz. So zeichnet sich die von der BASF Aktiengesellschaft vertriebene UV-Filtersubstanz Uvinul®T150 (INCI: Ethylhexyl Triazone) durch hervorragende UV Absorptionseigenschaften aus, aber dieser UV-Filter lässt sich in kosmetischen Ölen nur begrenzt lösen und kann in einer Reihe von Formulierungen nur zu relativ geringen Anteilen eingearbeitet werden, wodurch der durch diesen Filter zu erreichende Schutzfaktor limitiert wird.

Daher besteht weiterhin Bedarf nach verbesserten Darreichungsformen schwerlöslicher organischer UV-Filter, die den Einsatz bzw. die Anwendung dieser UV-Filter in größerer Menge erlauben. Eine wasserlösliche oder dispergierte Darreichungsform existiert bisher für Ethylhexyl Triazone nicht. Die US 6 537 529 offenbart ein Verfahren zur Herstellung von Sonnenschutzzusammensetzungen, enthaltend ein Lösungsmittel- und UV-Filtersystem. Die Referenz bezieht sich ebenfalls auf die gleiche Aufgabe: die verbesserte Löslichkeit des UVFilters zur Verbesserung des UV-Schutzes einer Zusammensetzung verbessert werden kann. Geeignete Lösungsmittel in D4 sind die Emollienten, die den Amiden der Formel (I) entsprechen.

Jetzt wurde gefunden, dass organische UV-Filter mit einer Löslichkeit in Dicaprylyl Carbonate von weniger als 40% bei 20°C bis 25°C und einer Versuchsdauer von 24 Stunden; hervorragend in verkapselter Form eingesetzt werden können, wenn in der Kapsel ein Emollient enthalten ist, welches in der Lage ist, bei 25°C den schwerlöslichen organischen UV-Filter mit größer 40 Gew.-% zu lösen und der Formel I entspricht.

Ein erster Gegenstand der vorliegenden Erfindung sind daher UV-Filter-Kapseln, umfassend
eine polymere Hülle und

a) mindestens einen organischen UV-Filter mit einer Löslichkeit in Dicaprylyl Carbonate von weniger als 40% bei 20°C bis 25°C und einer Versuchsdauer von 24 Stunden; und

b) ein Emollient, welches in der Lage ist, bei Raumtemperatur mehr als 40 Gew.-% des organischen UV-Filters a) zu lösen, dadurch gekennzeichnet, dass das unter b) genannte Emollient einer Verbindung der Formel I entspricht

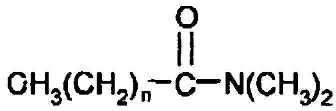 I

wobei n einer ganzen Zahl von 2 bis 12 entspricht.

Geeignete Kapseln können dabei Wände aus anorganischen oder organischen Polymeren aufweisen. Beispielsweise wird in US 6,242,099 B1 die Herstellung geeigneter Kapseln mit Wänden bzw. einer Hülle aus Chitin, Chitin-Derivaten oder polyhydroxylierten Polyaminen beschrieben. Erfindungsgemäß besonders bevorzugt einzusetzende Kapseln weisen Wände bzw. eine Hülle auf, die durch einen Sol-Gel-Prozeß, wie er in den Anmeldungen WO 00/09652, WO 00/72806, WO 00/71084, WO 03/39510 und WO 03/066209 beschrieben ist, erhalten werden können oder erhalten werden kann. Bevorzugt sind hier wiederum Kapseln, deren Wände oder Hülle aus Kieselgel (Silica; undefiniertes Siliciumoxidhydroxid) aufgebaut sind oder ist. Die Herstellung entsprechender Kapseln ist dem Fachmann beispielsweise aus den zitierten Patentanmeldungen bekannt, deren diesbezüglicher Inhalt ausdrücklich auch zum Gegenstand der vorliegenden Anmeldung gehört. Bevorzugt verwendete Kapseln bestehen demzufolge aus einer Hülle und einem Kern.

Das bevorzugt verwendete Verfahren zur Herstellung der erfindungsgemäßen UV-Filter-Kapseln erfolgt in drei Stufen: in Schritt a) wird eine öl-in-Wasser-Emulsion aus einer Mischung, enthaltend einen Sol-Gel-Vorläufer zur Herstellung der polymeren Hülle, mindestens einen UV-Filter mit einer Löslichkeit in Dicaprylyl Carbonate von weniger als 40% bei 20°C bis 25°C und einer Versuchsdauer von 24 Stunden; und ein Emollient, welches in der Lage ist, bei Raumtemperatur (20°C bis 25°C) mehr als 40 Gew.-% dieses organischen UV-Filters zu lösen und der Verbindung der Formel I entspricht

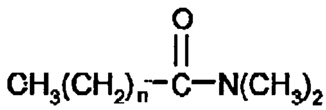 I

wobei n einer ganzen Zahl von 2 bis 12 entspricht, in einer wässrigen Lösung hergestellt,

in Schritt b) die in Schritt a) hergestellte Emulsion zu einer wässrigen Lösung mit einem pH von 2 bis 4, vorzugsweise von 3 bis 4, gemischt wird und gegebenenfalls werden

in Schritt c) Reaktionsprodukte aus dem Sol-Gel-Vorläufer abgetrennt.

Die wässrige Lösung aus Schritt b) dient in erster Linie zur Beschleunigung der zugrunde liegenden Kondensations-Polymerisations-Reaktion, die den Aufbau der Hülle bewirkt.

Nach einer angemessenen Reaktionszeit, in dem die Mischung auch erwärmt oder gekühlt oder auch der pH-Wert geändert werden kann, können die entstandenen Kapseln mit Mitteln, die dem Fachmann geläufig sind, isoliert werden. Beispielsweise können Sie zentrifugiert oder filtriert werden. Eine besonders bevorzugte Art der Isolierung ist die Sprühtrocknung. Das bedeutet, dass in Schritt c) neben der Abtrennung der Reaktionsprodukte aus dem Sol-Gel-Vorläufer, falls notwendig, die UV-Filter-Kapseln isoliert werden können.

In der Regel erhält man nach Schritt c) eine Dispersion oder Suspension, enthaltend die erfindungsgemäßen UV-Filter-Kapseln in einer Form, wie sie direkt in kosmetische oder dermatologische Zubereitungen eingesetzt werden kann. Auch eine Re-Suspension der isolierten Kapseln in beispielsweise deionisiertem Wasser oder in einem anderen Medium ist denkbar und für den Einsatz in die erfindungsgemäßen Zubereitungen verwendbar.

Die hydrophobe Lösung aus Schritt a) und auch die wässrigen Lösungen aus Schritt a) und b) können Tenside und/oder andere Additive enthalten, die diesen Prozess und/oder das Produkt verbessern oder auch stabilisieren können.

Als Sol-Gel-Vorläufer kann ein Metall oder Halbmetall-Alkoxid-Monomer, ein Metallester, Halbmetallester oder ein partiell hydrolysiertes und partiell kondensiertes Polymer oder eine Mischung davon verwendet werden. Geeignete und bevorzugte Sol-Gel-Vorläufer sind Verbindungen der Formel $M(R)_n(P)_m$, worin M ein Metall oder Halbmetall, bevorzugt Si, R ein hydrolysierbarer Subsituent und n eine ganze Zahl von 2 bis 4, P ein nichtpolymerisierbarer Substituent und m eine ganze Zahl von 0 bis 4 bedeutet oder ein partiell hydrolysiertes oder partiell kondensiertes Polymer davon oder irgend eine Mischung davon.

Insbesondere bevorzugt wird Tetraethylorthosilikat oder ein partiell hydrolysiertes oder partiell kondensiertes Polymer davon oder eine Mischung davon in dem zuvor beschriebenen Verfahren eingesetzt. Ganz besonders bevorzugt wird Tetraethylorthosilikat als Sol-Gel-Vorläufer eingesetzt.

Weitere Einzelheiten sind in den Ausführungsbeispielen offenbart.

Der UV-Filter kann eine Löslichkeit in Dicaprylyl Carbonate von weniger als 36%, 37%, 38%, oder 39% aufweisen.

In einer bevorzugten Ausführungsform handelt es sich bei dem unter a) genannten organischen UV-Filter um

ein Triazinderivat, Diarylbutadienderivat, Hydroxybenzophenonderivat und/oder Methylen bis-benzotriazolyl-tetramethylbutylphenolderivat.

Bei den Triazinderivaten sind die Verbindungen 2,4,6-Tris-[anilino-(p-carbo-2'-ethyl-1'-hexyloxy)]-1,3,5-triazin, Dioctylbutamidotriazon, Bis-Ethylhexyloxyphenol-methoxy-phenyltriazin, 2,4,6-tris(diethyl-4'-aminobenzalmalonate)-s-triazin, 2,4,6-tris(dimethyl-4'-amino-benzalmalonate-s-triazin, 2,4,6-tris(diiso-propyl-4-aminobenzal-malonate)-s-triazin, 2,4,6-tris[3'-benzotriazol-2-yl)-2'-hydroxy-5'-methyl)phenyl-amino]-s-triazin und 2,4,6-tris[3'benzotriazol-2-yl)-2'-hydroxy-5'-tert-octyl)phenyl-amino]-s-triazin bevorzugt. Besonders bevorzugt sind dabei die Triazinderivate 2,4,6-Tris-[anilino-(p-carbo-2'-ethyl-1'-hexyloxy)]-1,3,5-triazin (Uvinul®T150, BASF Aktiengesellschaft, nach INCI Ethylhexyl Triazone) Dioctylbutamidotriazon (UV-Sorb-HEB®, 3V Sigma) und Bis-Ethylhexyloxyphenol-methoxyphenyltriazin (Anisotriazine oder Tinosorb®S, Ciba-Geigy). Ganz besonders bevorzugt sind die Verbindungen 2,4,6-Tris-[anilino-(p-carbo-2'-ethyl-1'-hexyloxy)]-1,3,5-triazin (Uvinul®T150, BASF Aktiengesellschaft,) und/oder Dioctylbutamido-triazon (UV-Sorb-HEB®, 3V Sigma).

Weitere Triazinderivate können den Patentanmeldungen EP-A 0796851, EP-A 0087098 und EP-A 0850935 entnommen werden.

Bei den Diarylbutadienderivaten sind die 4,4'-Diarylbutadiene der Formel II,

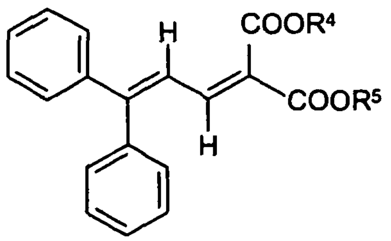

II

,

wobei $R^4$ und $R^5$ unabhängig voneinander H, $C_1$-$C_{20}$-Alkyl, $C_3$-$C_{10}$-Cycloalkyl oder $C_3$-$C_{10}$-Cycloalkenyl bedeuten, bevorzugt. Besonders bevorzugt ist die Verbindung 1,1-Dicarboxy(2',2'-dimethylpropyl)-4-4-diphenylbutadien. Die genannten 4,4'-Diarylbutadiene sind als solche bekannt und ihre Struktur und Herstellung sind in den Patentanmeldungen EP 0967200 und EP 916 335 beschrieben, auf deren Inhalt hiermit ausdrücklich Bezug genommen wird.

Bei den Hydroxybenzophenonen sind die Verbindungen der allgemeinen Formel III,

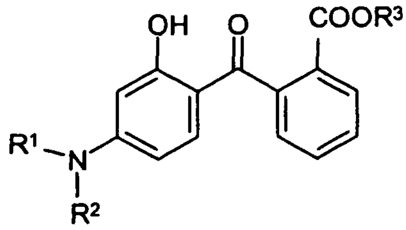

III

,

wobei $R^1$ und $R^2$ unabhängig voneinander H, $C_1$-$C_{20}$-Alkyl, $C_3$-$C_{10}$-Cycloalkyl oder $C_3$-$C_{10}$-Cycloalkenyl bedeuten, wobei die Substituenten $R^1$ und $R^2$ gemeinsam mit dem Stickstoffatom, an das sie gebunden sind, einen 5- oder 6-Ring bilden können und $R^3$ $C_1$-$C_{20}$-Alkyl bedeutet, bevorzugt. Besonders bevorzugt ist der 2-(4-N,N-Diethylamino-2-hydroxybenzoyl)-benzoesäure-hexylester (Uvinul®A Plus, BASF Aktiengesellschaft). Weiter Beispiele für Hydroxybenzophenone und deren Herstellung können der Deutschen Patentanmeldung DE-A 11917906 entnommen werden, auf deren Inhalt hiermit ausdrücklich Bezug genommen wird.

Als Alkylreste für Substituenten der Formeln II oder III seien verzweigte oder unverzweigte $C_1$-$C_{20}$-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dime-thylbutyl, 2,3-Dimethylbutyl, 3,3-Dimethylbutyl, 1-Ethylbutyl, 2-Ethylbutyl, 1,1, 2-Trimethylpropyl, 1,2,2-Trimethylpropyl, 1-Ethyl-1-methylpropyl, 1-Ethyl-2-methylpropyl, n-Heptyl, n-Octyl, 2-Ethylhexyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl genannt.

Als Alkenylreste für Substituenten der Formeln II oder III seien verzweigte oder unverzweigte $C_2$-$C_{10}$-Alkenylketten, bevorzugt Vinyl, Propenyl, Isopropenyl, 1-Butenyl, 2-Butenyl, 1-Pentenyl, 2-Pentenyl, 2-Methyl-1-butenyl, 2-Methyl-2-butenyl, 3-Methyl-1-butenyl, 1-Hexenyl, 2-Hexenyl, 1-Heptenyl, 2-Heptenyl, 1-Octenyl oder 2-Octenyl genannt.

Als Cycloalkylreste für Substituenten der Formeln II oder III seien bevorzugt verzweigte oder unverzweigte $C_3$-$C_{10}$-Cycloalkylketten wie so Cyclopropyl, Cyclobutyl, Cyclopentyl, Cyclohexyl, Cycloheptyl, 1-Methylcyclopropyl, 1-Ethylcyclopropyl, 1-Propylcyclo-propyl, 1-Butylcyclopropyl, 1-Pentylcyclopropyl, 1-Methyl-1-Butylcyclopropyl, 1,2-Dimethylcyclypropyl, 1-Methyl-2-Ethylcyclopropyl, Cyclooctyl, Cyclononyl oder Cyclodecyl genannt.

Als Cycloalkenylreste für Substituenten der Formeln II oder III seien bevorzugt verzweigte oder unverzweigte, $C_3$-$C_{10}$-Cycloalkenylketten mit einer oder mehreren Doppelbindungen wie Cyclopropenyl, Cyclobutenyl, Cyclopentenyl, Cyclopentadienyl, Cyclohexenyl, 1,3-Cyclohexadienyl, 1,4-Cyclohexadienyl, Cycloheptenyl, Cycloheptatrienyl, Cyclooctenyl, 1,5-Cyclooctadienyl, Cyclooctatetraenyl, Cyclononenyl oder Cyclodecyl genannt.

Bei den Methylen-bis-benzotriazolyl-tetramethylbutylphenolderivaten ist 2,2'-Methylenbis[6-(2H-benzotriazol-2-yl)-4-(1,1,3,3-tetramethylbutyl)phenol] (Tinosorb®M, Ciba-Geigy) bevorzugt. Diese Verbindungen sind auch in der FR 2440933 beschrieben, auf deren Inhalt hiermit ausdrücklich Bezug genommen wird.

In einer bevorzugten Ausführungsform enthalten die erfindungsgemäßen UV-Filter-Kapseln Mischungen der vorstehend genannten UV-Filter a).

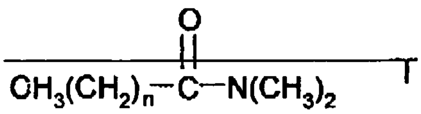

I

Bei den Verbindungen der Formel I steht die Zahl "n" bevorzugt für eine ganze Zahl von 3 bis 11, bevorzugt 4 bis 10, besonders bevorzugt 5 bis 9, am meisten bevorzugt 6 bis 8. In einer bevorzugten Ausführungsform handelt es sich bei der Verbindung der allgemeinen Formel I um N,N-Dimethyldecanamid (CAS Nr. 14433-76-2), ganz besonders bevorzugt um Spectrasolv DMDA (Jiangsu Feixang Chemicals).

Spectrasolv DMDA ist insbesondere ein hervorragendes Emollient für Ethylhexyl Triazone, da dessen Löslichkeit bei Raumtemperatur (20°C bis 25°C) bei ca. 55 Gew.-% liegt. Aufgrund dieser hervorragenden Löslichkeit gelingt es erstmals, UV-Filter-Kapseln herzustellen, deren Gehalt an Ethylhexyl Triazone sich deutlich von den Gehättern abhebt, die für die bisher am Markt befindlichen guten Lösungsmittel für Ethylhexyl Triazone erzielt werden. Eine Aufstellung diesbezüglich wird in den Ausführungsbeispielen gegeben.

Die erfindungsgemäßen UV-Filter-Kapseln enthalten dabei üblicherweise 1 bis 90 Gew.-%, vorzugsweise 5 bis 70 Gew.-% und insbesondere bevorzugt 40 bis 60 Gew.-% an dem organischen UV-Filter a), vorzugsweise im Kern, d.h. umschlossen von der polymeren Hülle. Bevorzugt besteht der Kern der erfindungsgemäßen UV-Filter-Kapseln zu 1 bis 90 Gew.-%, vorzugsweise zu 5 bis 70 Gew.-% und insbesondere bevorzugt zu 40 bis 60 Gew.-% aus dem mindestens einen organischen UV-Filter a), wobei erfindungsgemäß der weitere Kapselinhalt üblicherweise aus dem Emollient der Formel I und gegebenenfalls weiteren Hilfsstoffen, die die Löslichkeit des organischen UV-Filters weiter erhöhen oder stabilisieren, besteht.

Bevorzugt besteht daher der Kapselinhalt der erfindungsgemäßen Kapseln, d.h. der Kern, aus dem mindestens einen organischen UV-Filter a) und dem Emollient der Formel I, Mischungen von organischen UV-Filtern a), wie zuvor beschrieben und dem Emollient der Formeln I oder generell Mischungen mindestens eines organischen UV-Filters a) und löslicher organischer UV-Filter in dem Emollient der Formel I.

Weitere bevorzugte Kombinationen sind in den Ansprüchen offenbart.

Ganz besonders bevorzugt besteht der Kern aus Ethylhexyl Triazone und N,N-Dimethyldecanimid.

Die erfindungsgemäßen UV-Filter-Kapseln sind weiter dadurch gekennzeichnet, dass der Kern oder Kapselinhalt der UV-Filter-Kapsel den unter a) genannten UV-Filter und das unter b) genannte Emollient im Gewichtsprozentverhältnis 10:90 bis 90:10, vorzugsweise im Gewichtsprozentverhältnis 30:70 bis 70:30, besonders bevorzugt im Gewichtsprozentverhältnis von 40:60 bis 60:40 oder ganz besonders bevorzugt im Gewichtsprozentverhältnis von 50:50, enthält oder daraus besteht.

Im Einzelnen ergeben sich durch die Verkapselung folgende Vorteile:

- Die Hydrophilie der Kapselwand (oder synonym dazu der polymeren Hülle) kann unabhängig von der Löslichkeit des UV-Filters eingestellt werden. Deshalb können die organischen UV-Filter a) in rein wässrige Formulierungen eingearbeitet werden. Hierdurch kann der organische organische UV-Filter a) sowohl in der Ölals auch in der Wasserphase der kosmetischen oder dermatologischen Zubereitung als Endprodukt eingearbeitet werden. Dadurch lässt sich der Gesamtgehalt in kosmetischen Formulierungen steigern.
- Liegt ein schwerlöslicher UV-Filter in der organischen Phase vor und der erfindungsgemäß verkapselte UV-Filter a) in der wässrigen Phase vor, so wird ein sogenannter "boost-Effekt" beobachtet. Insbesondere tritt eine Synergle auf oder mit anderen Worten ein "boost", wenn der gleiche schwerlösliche UV-Filter in der organischen Phase und verkapselt in der Wasserphase vorliegt, dokumentierbar durch in vivo SPF-Werte von Formulierungen ohne erfindungsgemäß verkapselten UV-Filter a) und mit diesem.

- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.
- Allgemein können durch Verkapselung einzelner UV-Filter oder anderer Inhaftstoffe Formulierungsprobleme, die durch Wechselwirkung einzelner Formulierungsbestandteile untereinander entstehen, wie Kristallisationsvorgänge, Ausfällungen und Agglomeratbildung vermieden werden, da die Wechselwirkung unterbunden wird.
- Die gezielte Auswahl des besonderen Emollients, insbesondere der Auswahl von N,N-Dimethyldecanamid ((Spectrasolv DMDA) ermöglicht, dass die Löslichkeit des organischen UV-Filters a), wie zuvor beschrieben, insbesondere von Ethylhexyl Triazone, während der Verkapselung konstant bleibt, dass der UV-Filter nicht kristallisiert und damit eine hohe Beladung der Kapsel möglich wird. Das beschriebene System ist weiterhin temperaturstabil, so dass auch während der möglichen Temperaturschwankungen bei der Herstellung, eine stabile Kapsel entsteht.
- Der erfindungsgemäß verkapelte UV-Filter a) einsetzbar in der Wasserphase einer Zubereitung kann die in der Wasserphase üblichen wässrigen UV-Filter, wie beispielsweise Phenylbenzimidazolsulfonsäure, vorteilhaft ersetzen, da hierbei die notwendige pH-Regulierung entfällt, um zu stabilen Zubereitungen zu gelangen.

Bevorzugt liegt der Anteil der erfindungsgemäßen UV-Filter-Kapseln, wie zuvor beschrieben, in einer Dispersion bei 5 bis 80 Gew.-%, besonders bevorzugt bei 30 bis 70 Gew.-%, ganz besonders bevorzugt bei 35 bis 45 Gew.-%, bezogen auf die Gesamtmenge der Dispersion. In den Ausführungsbeispielen wird der Wassergehalt der beschriebenen Dispersionen bei ca. 60 Gew.-% angegeben.

Ein weiterer Gegenstand der Erfindung ist die Verwendung der erfindungsgemäßen UV-Filter-Kapseln, wie zuvor beschrieben, oder einer Dispersion, enthaltend die UV-Filter-Kapseln, wie zuvor und nachstehend beschrieben, zur Herstellung einer Zubereitung, insbesondere einer kosmetischen oder dermatologischen Zubereitung.

Ein weiterer Gegenstand der Erfindung ist eine Dispersion oder eine Suspension, enthaltend die erfindungsgemäßen UV-Filter-Kapseln, wie zuvor beschrieben. Bevorzugt ist diese Dispersion eine wässrige Dispersion, d.h. das Dispergiermedium ist Wasser. Weitere Dispergiermedien können auch beliebige andere geeignete Substanzen sein. Besonders geeignet sind mehrwertige Alkohole, z.B. Glycerin oder 1,2-Propandiol. Das Dispergiermedium kann auch eine geeignete Mischung sein, z.B. ein Glycerin-Wasser-Gemisch in beliebigem Verhältnis.

Es handelt sich hierbei um Vordispersionen, die sich zum einen selbst direkt als kosmetische oder dermatologische Zubereitung eignen und zum anderen die Herstellung solcher Zubereitungen, die einen Träger enthalten, erleichtern können. Bevorzugt ist der Träger für topische Zwecke geeignet, d.h. für eine örtliche, insbesonder oberflächlich auftragbare Form, geeignet.

Die erfindungsgemäßen Vordispersionen können in die Wasserphase einer jeden Zubereitung, insbesondere in eine kosmetische oder dermatologische Zubereitung, eingearbeitet werden.

Ein weiterer Gegenstand der Erfindung ist demzufolge eine Zubereitung enthaltend mindestens einen organischen UV-Filter a) und mindestens einen geeigneten Träger, insbesondere einen für topische Zwecke geeigneten Träger, dadurch gekennzeichnet, dass mindestens ein Teil des organischen UV-Filters a) verkapselt vorliegt und zwar in verkapselter Form, wie für die erfindungsgemäßen UV-Filter-Kapseln beschrieben.

Besonders bevorzugt zur Erzielung verbesserter Synergien hinsichtlich des Lichtschutzfaktors sind solche erfindungsgemäßen Zubereitungen, die eine Ölphase und eine wässrige Phase enthalten, wobei die erfindungsgemäßen UV-Filter-Kapseln in der wässrigen Phase vorliegen, und gleichzeitig in der Ölphase mindestens ein weiterer öllöslicher oder der bereits in den Kapseln befindliche UV-Filter a), insbesondere der UV-Filter a), wie beispielsweise 2,4,6-Tris[anilino-(p-carbo-2'-ethyl-1'-hexyloxy)]-1,3,5-triazin, 2-(4-Diethylamino-2-hydroxybenzoyl)-benzoesäurehexylester, 1,1-Dicarboxy(2',2'-dimethylpropyl)-4-4-diphenylbutadien oder Bis-Ethylhexyloxyphenol-methoxyphenyltriazin, vorhanden ist.

Die Zubereitung kann die genannten notwendigen oder optionalen Bestandteile bzw. Inhaltsstoffe umfassen oder enthalten, daraus im wesentlichen oder daraus bestehen. Alle Verbindungen oder Komponenten, die in den Zubereitungen verwendet werden können, sind entweder bekannt und käuflich erwerbbar oder können nach bekannten oder hier beschriebenen Verfahren synthetisiert werden.

Im Sinne der Erfindung wird der Begriff Zubereitung gleichbedeutend mit dem Begriff Formulierung oder Mittel verwendet.

Hautkosmetische, haarkosmetische, dermatologische, hygienische oder pharmazeutische Mittel, Zubereitungen und/oder Formulierungen zur topischen Anwendung auf Haut oder Haar sind geeignet (i) zur Prävention von Schädigungen der menschlichen Haut und/oder menschlicher Haare, (ii) zur Behandlung von bereits aufgetretenen Schädigungen der menschlichen Haut und/oder menschlicher Haare, (iii) zur Pflege der menschlichen Haut und/oder menschlicher Haare, (iv) zur Verbesserung des Hautgefühls (sensorische Eigenschaften). Explizit umfaßt sind Mittel zur dekorativen Kosmetik. Ferner umfaßt sind Mittel zur Hautpflege, bei denen der pharmazeutisch dermatologische Anwendungszweck unter Mitberücksichtigung kosmetischer Gesichtspunkte erreicht wird. Derartige Mittel oder Zubereitungen werden zur Unterstützung, Vorbeugung und Behandlung von Hauterkrankungen eingesetzt und können neben dem kosmetischen Effekt eine biologische Wirkung entfalten. Besonders bevorzugt handelt es sich bei den erfindungsgemäßen Zubereitungen um Zubereitungen zum Schutz der Haut vor Schädigungen durch Sonnenlicht, im speziellen durch UV-B- (280 bis 320 nm) und UV-A-Strahlung (>320 nm). Diese enthalten in einem kosmetisch verträglichen Medium geeignete Hilfs- und Zusatzstoffe, welche im Hinblick auf das spezielle Anwendungsgebiet gewählt werden. Derartige Hilfs- und Zusatzstoffe sind dem Fachmann geläufig und können z.B. Handbüchern der Kosmetik, beispielsweise Schrader, Grundlagen und Rezepturen der Kosmetika, Hüthig Verlag, Heidelberg, 1989, ISBN 3-7785-1491-1, oder Umbach, Kosmetik: Entwicklung, Herstellung und Anwendung kosmetischer Mittel, 2. erweiterte Auflage, 1995, Georg Thieme Verlag, ISBN 3 13 712602 9, entnommen werden.

Daher gibt es erfindungsgemäß zur Herstellung der Zubereitungen, insbesondere kosmetischer oder dermatologischer Zubereitungen, enthaltend erfindungsgemäße Kapseln, beispielsweise folgende Verfahren:

- Ein Verfahren zur Herstellung einer Zubereitung, insbesondere einer kosmetischen oder dermatologischen Zubereitung mit Lichtschutzeigenschaften, ist dadurch gekennzeichnet, dass die erfindungsgemäßen Kapseln mit weiteren Inhaltsstoffen vermischt werden.
- Ein Verfahren zur Herstellung einer Zubereitung, insbesondere einer kosmetischen oder dermatologischen Zubereitung mit Lichtschutzeigenschafte, ist dadurch gekennzeichnet, dass eine Vordispersion, enthaltend die erfindungsgemäßen Kapseln, mit weiteren Inhaltsstoffen vermischt wird.
- Ein besonderes Verfahren zur Herstellung einer Zubereitung, insbesondere einer kosmetischen oder dermatologischen Zubereitung, in Form einer Öl-in-Wasser-Emulsion ist dadurch gekennzeichnet, dass die zuvor beschriebene Vordispersion mit einem Öl emulgiert wird.

Dabei sind die Kapseln in erfindungsgemäßen Formulierungen vorzugsweise in solchen Mengen enthalten, die gewährleisten, dass die verkapselten UV-Filter in wirksamen Mengen in der Formulierung vorliegen.

Bevorzugt ist der Einsatz der erfindungsgemäßen UV-Filter-Kapseln in den Zubereitungen von 1 bis 40 Gew.-%, besonders bevorzugt von 5 bis 30 Gew.-%, ganz besonders bevorzugt von 5 bis 20 Gew.-%, bezogen auf die Gesamtmenge der Zubereitung.

Die Größe der erfindungsgemäßen UV-Filter-Kapseln können von 0,001 bis 20,0 μm variieren, wobei die mittlere Teilchengröße d(0,50), bestimmt über eine Partikelgrössenbestimmung mittels Laserbeugung nach ISO/DIS 13320, in der Regel bei 200 bis 5000 nm, bevorzugt bei 400 bis 1500 nm liegt. Die Methode wird im Ausführungsteil beschrieben.

Die Zubereitungen, insbesondere die kosmetischen oder dermatologischen Zubereitungen, bevorzugt mit Lichtschutzeigenschaften, können in verschiedenen Formen vorliegen. So können sie z. B. eine Lösung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen.

Bevorzugt ist es dabei insbesondere, wenn es sich bei der kosmetischen oder dermatologischen Zubereitung um eine wässrige Zubereitung, insbesondere ein Gel, oder eine Emulsion, insbesondere eine Öl-in-Wasser-Emulsion (O/W-Emulsion) handelt, da bei der Herstellung derartiger Zubereitungen die Vorteile der erfindungsgemäßen Formulierungen in besonderer Weise zur Geltung kommen.

Emulsionen, enthaltend die oben beschriebene erfindungsgemäße Formulierung in der bzw. als Wasserphase sind daher ein weiterer Gegenstand der vorliegenden Erfindung. Insbesondere bevorzugt sind dabei Öl-in-Wasser-Emulsion (O/W-Emulsion).

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettlkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der

Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12}$-$_{15}$-Alkylbenzoat, Capryl-Caprinsäure-triglycerid, Dicaprylether.

Besonders vorteilhaft sind Mischungen aus $C_{12}$-$_{15}$-Alkylbenzoat und 2-Ethylhexylisostearat, Mischungen aus $C_{12\text{-}15}$-Alkylbenzoat und Isotridecylisononanoat sowie Mischungen aus $C_{12\text{-}15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann auch die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formuierung verwendet wird.

Als Emulgatoren können beispielsweise die bekannten W/O- und O/W-Emulgatoren verwendet werden. Es ist vorteilhaft, weitere übliche Co-emulgatoren in den erfindungsgemäßen bevorzugten O/W-Emulsionen zu verwenden.

Erfindungsgemäß vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen. Insbesondere bevorzugt sind: Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17),Polyethylenglycol(18)stearylether (Steareth-18), Polyethylenglycol(19)-stearylether (Steareth-19), Polyethylenglycol(20)stearylether (Steareth-20), Polyethylenglycol(12)isostearylether (Isosteareth-12), Polyethylenglycol(13)isostearylether (Isosteareth-13), Polyethylenglycol(14)-isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol-

col(18)isostearylether (Isosteareth-18), Polyethylenglycol(19)isostearylether (Isosteareth-19), Polyethylenglycol(20)-isostearylether (Isosteareth-20), Polyethylenglycol(13)cetylether (Ceteth-13), Polyethylenglycol(14)cetylether (Ceteth-14), Polyethylenglycol(15)-cetylether (Ceteth-15), Polyethylenglycol(16)cetylether (Ceteth-16), Polyethylenglycol(17)cetylether (Ceteth-17), Polyethylenglycol(18)-cetylether (Ceteth-18), Polyethylenglycol(19)cetylether (Ceteth-19), Polyethylen-glycol(20)cetylether (Ceteth-20), Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)-isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20), Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15), Polyethylenglycol(12)laurylether (Laureth-12), Polyethylenglycol(12)-isolaurylether (Isolaureth-12), Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)-cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20).

Es ist ferner von Vorteil, die Fettsäureethoxylate ausfolgender Gruppe zu wählen:

Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat,

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/cprinat, Polyethylenglycol-(20)glyceryloleat, Polyethylenglycol(20)glycerylisostearat, Polyethylenglycol(18)glyceryloleat(cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:

Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkhole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat oder PEG-30-dipolyhydroxystearat.

Erfindungsgemäß herzustellende kosmetische und dermatologische Zubereitungen enthalten außerdem vorteilhaft, wenngleich nicht zwingend, anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$), Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$, und insbesondere um mikronisiertes $TiO_2$.

Erfindungsgemäß können die kosmetischen und/oder dermatologischen Lichtschutzformulierungen wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Besonders bevorzugt werden erfindungsgemäß solche kosmetischen und dermatologischen Zubereitungen hergestellt, die in Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt hydrophobe anorganische Mikropigmente, enthalten.

Besonders bevorzugt sind solche UV-Filter, deren physiologische Unbedenklichkeit bereits nachgewiesen ist. Sowohl für UV-A wie auch UV-B-Filter gibt es aus der Fachliteratur bekannte Substanzen, z.B.

Benzylidenkampferderivate wie 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)anilinium methylsulfat (z.B. Mexoryl® SK) oder (2-Oxoborn-3-yliden)toluol-4-sulfonsäure (z.B. Mexoryl® SL),

Benzoyl- oder Dibenzoylmethane wie 1-(4-tert-Butylphenyl)-3-(4-methoxyphenyl)propan-1,3-dion (z.B. Eusolex® 9020) oder 4-Isopropyldibenzoylmethan (z.B. Eusolex® 8020),

Benzophenone wie 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40),

Methoxyzimtsäureester wie Methoxyzimtsäureoctylester (z.B. Eusolex® 2292), 4-Methoxyzimtsäureisopentylester, z.B. als Gemisch der Isomere (z.B. Neo Heliopan® E 1000),

Salicylatderivate wie 2-Ethylhexylsalicylat (z.B. Eusolex® OS), 4-Isopropylbenzylsalicylat (z.B. Megasol®) oder 3,3,5-Trimethylcyclohexylsalicylat (z.B. Eusolex® HMS),

4-Aminobenzoesäure und Derivate wie 4-Aminobenzoesäure, 4-(Dimethylamino)benzoesäure-2-ethylhexylester (z.B. Eusolex® 6007), ethoxylierter 4-Aminobenzoesäureethylester (z.B. Uvinul® P25),

Phenylbenzimidazolsulfonsäuren, wie 2-Phenylbenzimidazol-5-sulfonsäure sowie ihre Kalium-, Natrium- und Triethanolaminsalze (z.B. Eusolex® 232), 2,2-(1,4-Phenylen)-bisbenzimidazol-4,6-disulfonsäure bzw. deren Salze (z.B. Neoheliopan® AP) oder 2,2-(1,4-Phenylen)-bisbenzimidazol-6-sulfonsäure;
und weitere Substanzen wie

- 2-Cyano-3,3-diphenylacrylsäure-2-ethylhexylester (z.B. Eusolex® OCR),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX) und
- 2,4,6-Trianilino-(p-carbo-2'-ethylhexyl-1'-oxi)-1,3,5-triazin (z.B. Uvinul® T 150)
- 2-(4-Diethylamino-2-hydroxy-benzoyl)-benzoesäure hexylester (z.B. Uvinul®UVA Plus, Fa. BASF).

Die in der Liste aufgeführten Verbindungen sind nur als Beispiele aufzufassen. Selbstverständlich können auch andere UV-Filter verwendet werden.

Diese organischen UV-Filter werden in der Regel in einer Menge von 0,5 bis 10 Gewichtsprozent, vorzugsweise 1-8 Gew.-%, in kosmetische Formulierungen eingearbeitet.

Weitere geeignete organische UV-Filter sind z.B.

- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole®),
- 4,4'-[(6-[4-((1,1-Dimethylethyl)aminocarbonyl)phenylaminol-1,3,5-triazin-2,4-diyl)diimino]bis(benzoesäure-2-ethylhexylester) (z.B. Uvasorb® HEB),
- Dimethicone diethylbenzalmalonate (CAS-Nr. 207 574-74-1)
- 2,2'-Methylen-bis-(6-(2H-benzotriazol-2-yl)-4-(1,1,3,3-tetramethyl-butyl)phenol) (CAS-Nr. 103 597-45-1)
- 2,2'-(1,4-Phenylen)bis-(1 H-benzimidazol-4,6-disulfonsäure, Mononatriumsalz) (CAS-Nr. 180 898-37-7) und
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187

393-00-6).

- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),

Weitere geeignete UV-Filter sind auch Methoxyflavone ensprechend der Deutschen Patentanmeldung DE 10232595.

Organische UV-Filter werden in der Regel in einer Menge von 0,5 bis 20 Gewichtsprozent, vorzugsweise 1-15 Gew.-%, in kosmetische Formulierungen eingearbeitet.

Als anorganische UV-Filter sind solche aus der Gruppe der Titandioxide wie z.B. gecoatetes Titandioxid (z.B. Eusolex® T-2000, Eusolex®T-AQUA; Eusolex® T-AVO,), Zinkoxide (z.B. Sachtotec®), Eisenoxide oder auch Ceroxide denkbar. Diese anorganischen UV-Filter werden in der Regel in einer Menge von 0,5 bis 20 Gewichtsprozent, vorzugsweise 2-10 Gew.-%, in kosmetische Zubereitungen eingearbeitet.

Bevorzugte Verbindungen mit UV-filternden Eigenschaften sind 3-(4'-Methylbenzyliden)-dl-kampfer, 1-(4-tert-Butylphenyl)-3-(4-methoxy-phenyl)-pro-pan-1,3-dion, 4-Isopropyldibenzoylmethan, 2-Hydroxy-4-meth-oxy-ben-zo--phenon, Methoxyzimtsäureoctylester, 3,3,5-Trimethyl-cyclo-hexylsali-cylat, 4-(Dimethylamino)benzoesäure-2-ethyl-hexylester, 2-Cyano-3,3-di-phenyl-acrylsäure-2-ethylhexylester, 2-Phenyl-benzimidazol-5-sulfonsäure sowie ihre Kalium-, Natrium- und Triethanol-aminsalze.

Neben den hier beschriebenen Verbindungen können die erfindungsgemäßen Zubereitungen auch mindestens einen Photostabilisator, vorzugsweise entsprechend der Formel I

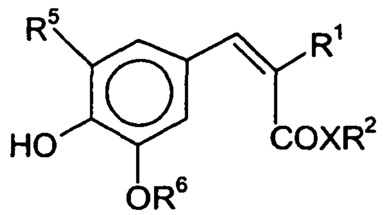

I,

wobei

$R^1$ ausgewählt ist aus $-C(O)CH_3$, $-CO_2R^3$, $-C(O)NH_2$ and $-C(O)N(R^4)_2$;
X is O or NH;
$R^2$ steht für einen linearen oder verzweigten $C_{1-30}$-Alkylrest;
$R^3$ steht für einen linearen oder verzweigten $C_{1-20}$-Alkylrest,
alle $R^4$ unabhängig voneinander stehen für H oder lineare oder verzweigte $C_{1-8}$-Alkylreste
$R^5$ steht für H, einen linearen oder verzweigten $C_{1-8}$-Alkylrest oder einen linearen oder verzweigten $-O-C_{1-8}$-Alkylrest und
$R^6$ steht für einen $C_{1-8}$-Alkylrest,

wobei es sich bei dem Photostabilisator insbesondere bevorzugt um 2-(4-Hydroxy-3,5-dimethoxy-benzyliden)-malonsäure-bis-(2-ethyl-hexyl)ester handelt, enthalten. Entsprechende Photostabilisatoren, ihre Herstellung und Verwendung sind in der Internationalen Patentanmeldung WO 03/007906 beschrieben, deren Offenbarung ausdrücklich auch zum Gegenstand der vorliegenden Anmeldung gehört. Die erwähnten Verbindungen sind auch Antioxidantien.

Ferner ist es möglich und vorteilhaft, die erfindungsgemäßen Zubereitungen mit Antioxidationsmitteln zu kombinieren. Eine solche Kombination zeigt dann sowohl Schutzwirkung als Antioxidationsmittel als auch vor Verbrennungen durch UV-Strahlung. Somit kann man auch eine schützende Wirkung gegen oxidativen Stress bzw. gegen die Einwirkung von Radikalen erzielen.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall-) Chelatoren, (z.B. α-Hydroxyfettsäuren, Palmitinsäure, Phytinsäure, Lactoferrin), α-Hydroxysäuren (z.B. Citronensäure,

Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, $ZnSO_4$), Selen und dessen Derivate (z.B. Selenmethionin), Stilbene und deren Derivate (z.B. Stilbenoxid, trans-Stilbenoxid).

Geeignete Antioxidantien sind auch Verbindungen der allgemeinen Formeln A oder B

A 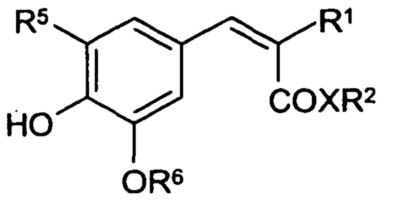 oder 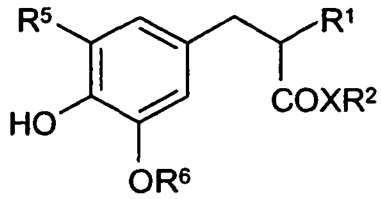 B ,

worin

$R^1$ aus der Gruppe $-C(O)CH_3$, $-CO_2R^3$, $-C(O)NH_2$ und $-C(O)N(R^4)_2$ ausgewählt werden kann,
X O oder NH,
$R^2$ lineares oder verzweigtes Alkyl mit 1 bis 30 C-Atomen,
$R^3$ lineares oder verzweigtes Alkyl mit 1 bis 20 C-Atomen,
$R^4$ jeweils ungabhängig voneinander H oder lineares oder verzweigtes Alkyl mit 1 bis 8 C-Atomen,
$R^5$ lineares oder verzweigtes Alkyl mit 1 bis 8 C-Atomen oder lineares oder verzweigtes Alkoxy mit 1 bis 8 C-Atomen und
$R^6$ lineares oder verzweigtes Alkyl mit 1 bis 8 C-Atomen bedeutet, vorzugsweise Derivate der 2-(4-Hydroxy-3,5-dimethoxybenzyliden)-malonsäure und/oder 2-(4-Hydroxy-3,5-dimethoxybenzyl)-malonsäure, besonders bevorzugt 2-(4-Hydroxy-3,5-dimethoxybenzyliden)-malonsäurebis-(2-ethylhexyl)ester (z.B. Oxynex® ST Liquid) und/oder 2-(4-Hydroxy-3,5-dimethoxybenzy)-malonsäure-bis-(2-ethylhexyl)ester (z.B. RonaCare® AP). Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004).

Die erfindungsgemäßen Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin $B_1$), Riboflavin (Vitamin $B_2$), Nicotinsäureamid, Vitamin C (Ascorbinsäure), Vitamin D, Ergocalciferol (Vitamin $D_2$), Vitamin E, DL-α-Tocopherol, Tocopherol-E-Acetat, Tocopherolhydrogensuccinat, Vitamin $K_1$, Esculin (Vitamin P-Wirkstoff), Thiamin (Vitamin $B_1$), Nicotinsäure (Niacin), Pyridoxin, Pyridoxal, Pyridoxamin, (Vitamin $B_6$), Panthothensäure, Biotin, Folsäure und Cobalamin (Vitamin $B_{12}$) in den erfindungsgemäßen kosmetischen Zubereitungen enthalten, insbesondere bevorzugt Vitamin-A-Palmitat, Vitamin C und dessen Derivaten, DL-α-Tocopherol, Tocopherol-E-Acetat, Nicotinsäure, Pantothensäure und Biotin.

Die erfindungsgemäßen Zubereitungen können darüber hinaus weitere übliche hautschonende oder hautpflegende Wirkstoffe enthalten. Dies können prinzipiell alle den Fachmann bekannten Wirkstoffe sein, wie insbesondere Flavon-Derivate, Chromon-Derivate, kompatible Solute und andere Wirkstoffe.

Es kann bevorzugt sein, wenn die erfindungsgemäße Zubereitung mindestens ein Repellent enthält, wobei das Repellent vorzugsweise ausgewählt aus N,N-Diethyl-3-methylbenzamid, 3-(Acetyl-butyl-amino)-propionsäure Ethylester, Dimethylphthalat, Butopyronoxyl, 2,3,4,5-bis-(2-Butylen)-tetrahydro-2-furaldehyd, N,N-Caprylsäurediethylamid, N,N-Diethylbenzamid, o-Chlor-N,N-diethylbenzamid, Dimethylcarbat, Di-n-propylisocinchomeronat, 2-Ethylhexan-1,3-diol, N-Octyl-bicyclohepetendiecarboximid, Piperonyl-butoxid, 1-(2-Methylpropyloxycarbonyl)-2-(hydroxyethyl)-piperidin oder Mischungen davon, wobei es insbesondere bevorzugt ausgewählt ist aus N,N-Diethyl-3-methylbenzamid, 3-(Acetyl-butyl-amino)-propionsäure-ethylester 1-(2-Methylpropyloxycarbonyl)-2-(hydroxyethyl)-piperidin oder Mischungen davon.

Bei den erfindungsgemäßen Zubereitungen, die Repellentien enthalten, handelt es sich dabei vorzugsweise um Insektenabwehrmittel. Insektenabwehrmittel werden in Form von Lösungen, Gelen, Stiften, Rollern, Pump-Sprays und Aerosol-Sprays angeboten, wobei Lösungen und Sprays den Hauptteil der im Handel erhältlichen Produkte bilden. Basis für diese beiden Produktformen sind meist alkoholische bzw. wässrigalkoholische Lösungen unter Zusatz fettender Substanzen und leichter Parfümierung.

Als Flavon-Derivate werden erfindungsgemäß Flavonoide und Coumaranone verstanden. Als Flavonoide werden erfindungsgemäß die Glykoside von Flavanonen, Flavonen, 3-Hydroxyflavonen (= Flavonolen), Auronen, Isoflavonen und Rotenoiden aufgefaßt [Römpp Chemie Lexikon, Band 9, 1993]. Im Rahmen der vorliegenden Erfindung werden hierunter jedoch auch die Aglykone, d.h. die zuckerfreien Bestandteile, und die Derivate der Flavonoide und der Aglykone verstanden. Weiterhin wird im Rahmen der vorliegenden Erfindung unter dem Begriff Flavonoid auch Anthocyanidin (Cyanidin) verstanden. Im Rahmen der vorliegenden Erfindung werden unter Coumaranonen auch deren Derivate verstanden.

Bevorzugte Flavonoide leiten sich von Flavanonen, Flavonen, 3-Hydroxyflavonen, Auronen und Isoflavonen, insbesondere von Flavanonen, Flavonen, 3-Hydroxyflavonen und Auronen, ab.

Die Flavonoide sind vorzugsweise ausgewählt aus folgenden Verbindungen: 4,6,3',4'-Tetrahydroxyauron, Quercetin, Rutin, Isoquercetin, Eriodictyol, Taxifolin, Luteolin, Trishydroxyethylquercetin (Troxequercetin), Trishydroxyethylrutin (Troxerutin), Trishydroxyethylisoquercetin (Troxeisoquercetin), Trishydroxyethylluteolin (Troxeluteolin), α-Glycosylrutin, Tilirosid sowie deren Sulfaten und Phosphaten. Unter den Flavonoiden sind als erfindungsgemäße Aktivstoffe insbesondere Rutin, Tilirosid, α-Glycosylrutin und Troxerutin bevorzugt.

Unter den Phenolen mit antioxidativer Wirkung sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydoxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH-Gruppen in 3'4'- oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers, I.M.C.M. Rietjens; Free Radical Biology&Medicine 2001, 31(7), 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Geeignete Antioxidantien sind weiter Verbindungen der Formel II

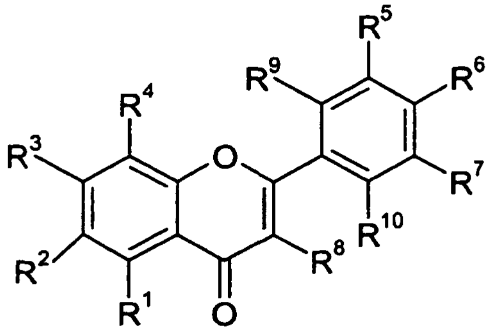

II

wobei $R^1$ bis $R^{10}$ gleich oder verschieden sein können und ausgewählt sind aus

- H
- OR"
- geradkettigen oder verzweigten $C_1$- bis $C_{20}$-Alkylgruppen,
- geradkettigen oder verzweigten $C_3$- bis $C_{20}$-Alkenylgruppen,
- geradkettigen oder verzweigten $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können,

- wobei alle $OR^{11}$ unabhängig voneinander stehen für

  - OH
  - geradkettige oder verzweigte $C_1$- bis $C_{20}$-Alkyloxygruppen,
  - geradkettigen oder verzweigten $C_3$- bis $C_{20}$-Alkenyloxygruppen,
  - geradkettigen oder verzweigten $C_1$- bis $C_{20}$-Hydroxyalkoxygruppen, wobei die Hydroxygruppe(n) an ein primäre oder sekundäre Kohlenstoffatome der Kette gebunden sein können und weiter die Alkylkette auch durch Sauerstoff unterbrochen sein kann, und/oder
  - $C_3$- bis $C_{10}$-Cycloalkyloxygruppen und/oder $C_3$- bis $C_{12}$-Cycloalkenyloxygruppen, wobei die Ringe jeweils auch durch $-(CH_2)_n$-Gruppen mit n = 1 bis 3 überbrückt sein können und/oder,
  - Mono- und/oder Oligoglycosylreste, mit der Maßgabe, dass mindestens 4 Reste aus $R^1$ bis $R^7$ stehen für OH und dass im Molekül mindestens 2 Paare benachbarter Gruppen -OH vorliegen,

- oder $R^2$, $R^5$ und $R^6$ für OH und die Reste $R^1$, $R^3$, $R^4$ und $R^{7-10}$ für H stehen,

wie sie in der Deutschen Patentanmeldung DE-A-10244282 beschrieben sind.

Unter den Coumaranonen ist 4,6,3',4'-Tetrahydroxybenzylcoumaranon-3 bevorzugt.

Unter Chromon-Derivaten werden vorzugsweise bestimmte Chromen-2-onDerivate, die sich als Wirkstoffe zur vorbeugenden Behandlung von menschlicher Haut und menschlicher Haare gegen Alterungsprozesse und schädigende Umwelteinflüssen eignen, verstanden. Sie zeigen gleichzeitig ein niedriges Irritationspotential für die Haut, beeinflussen die Wasserbindung in der Haut positiv, erhalten oder erhöhen die Elastizität der Haut und fördern somit eine Glättung der Haut. Diese Verbindungen entsprechen vorzugsweise der Formel III

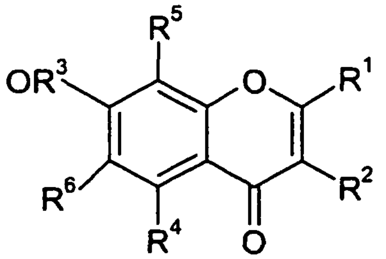

III

wobei $R^1$ und $R^2$ gleich oder verschieden sein können und ausgewählt sind aus

- H, $-C(=O)-R^7$, $-C(=O)-OR^7$,
- geradkettigen oder verzweigten $C_1$- bis $C_{20}$-Alkylgruppen,
- geradkettigen oder verzweigten $C_3$- bis $C_{20}$-Alkenylgruppen, geradkettigen oder verzweigten $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder

  - $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können,
    $R^3$ steht für H oder geradkettige oder verzweigte $C_1$- bis $C_{20}$-Alkylgruppen,
    $R^4$ steht für H oder $OR^8$,
    $R^5$ und $R^6$ gleich oder verschieden sein können und ausgewählt sind aus

- -H, -OH,
- geradkettigen oder verzweigten $C_1$- bis $C_{20}$-Alkylgruppen,
- geradkettigen oder verzweigten $C_3$- bis $C_{20}$-Alkenylgruppen,
- geradkettigen oder verzweigten $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann und
  $R^7$ steht für H, geradkettige oder verzweigte $C_1$- bis $C_{20}$-Alkylgruppen, eine Polyhydroxy-Verbindung, wie vorzugsweise einen Ascorbinsäurerest oder glycosidische Reste und
  $R^8$ steht für H oder geradkettige oder verzweigte $C_1$- bis $C_{20}$-Alkylgruppen, wobei mindestens 2 der Substituenten $R^1$, $R^2$, $R^4$-$R^6$ verschieden von H sind oder mindestens ein Substituent aus $R^1$ und $R^2$ für $-C(=O)-R^7$ oder $-C(=O)-OR^7$ steht.

Der Anteil an einer oder mehreren Verbindungen ausgewählt aus Flavonoiden, Cromon-Derivaten und Coumaranonen in der erfindungsgemässen Zubereitung beträgt vorzugsweise von 0,001 bis 5 Gew.-%, besonders bevorzugt von 0,01 bis 2 Gew.-% bezogen auf die gesamte Zubereitung.

Besonders bevorzugte Wirkstoffe sind beispielsweise auch sogenannte kompatible Solute. Es handelt sich dabei um Substanzen, die an der Osmoregulation von Pflanzen oder Mikroorganismen beteiligt sind und aus diesen Organismen isoliert werden können. Unter den Oberbegriff kompatible Solute werden dabei auch die in der Deutschen Patentanmeldung DE-A-10133202 beschriebenen Osmolyte gefasst. Geeignete Osmolyte sind beispielsweise die Polyole, Methylamin-Verbindungen und Aminosäuren sowie jeweils deren Vorstufen. Als Osmolyte werden im Sinne der Deutschen Patentanmeldung DE-A-10133202 insbesondere Substanzen aus der Gruppe der Polyole, wie beispielsweise myo-Inositol, Mannitol oder Sorbitol und/oder einer oder mehrere der nachfolgend genannten osmolytisch wirksamen Stoffe verstanden: Taurin, Cholin, Betain, Phosphorylcholin, Glycerophosphorylcholine, Glutamin, Glycin, α-Alanin, Glutamat, Aspartat, Prolin, und Taurin. Vorstufen dieser Stoffe sind beispielsweise Glucose, Glucose-Polymere, Phosphatidylcholin, Phosphatidylinositol, anorganische Phosphate, Proteine, Peptide und Polyaminsäuren. Vorstufen sind z.B. Verbindungen, die durch metabolische Schritte in Osmolyte umgewandelt werden.

Vorzugsweise werden erfindungsgemäß als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N.-Acetylornithin, Trimethylamine-N-oxid Di-myoinositol-phosphat (DIP), cyclisches 2,3-diphosphoglycerat (cDPG), 1,1-Diglycerin-Phosphat (DGP), β-Mannosylglycerat (Firoin), β-Mannosylglyceramid (Firoin-A) oder/und Di-mannosyl-di-inositolphosphat (DMIP) oder ein optisches Isomer, Derivat, z.B. eine Säure, ein Salz oder Ester dieser Verbindungen oder Kombinationen davon eingesetzt.

Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure und deren Derivate zu nennen. Diese Verbindungen stabilisieren Enzyme und andere Biomoleküle in wässrigen Lösungen und organischen Lösungsmitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutische wirksame Peptide und Proteine z.B. t-PA können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Ups, Pflegecremes und Sonnenschutzpräparaten eingesetzt werden.

Dabei wird vorzugsweise eine Pyrimidincarbonsäure gemäß der unten stehenden Formel IV eingesetzt,

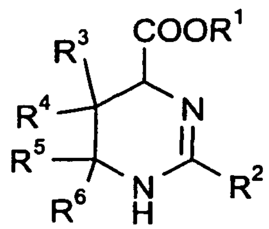

IV

worin $R^1$ ein Rest H oder C1-8-Alkyl, $R^2$ ein Rest H oder C1-4-Alkyl und $R^3$, $R^4$, $R^5$ sowie $R^6$ jeweils unabhängig voneinander ein Rest aus der Gruppe H, OH, $NH_2$ und C1-4-Alkyl sind. Bevorzugt werden Pyrimidincarbonsäuren eingesetzt, bei denen $R^2$ eine Methyl- oder eine Ethylgruppe ist und $R^1$ bzw. $R^5$ und $R^6$ H sind. Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) eingesetzt. Dabei enthalten die erfindungsgemäßen Zubereitungen derartige Pyrimidincarbonsäuren vorzugsweise in Mengen bis zu 15 Gew.-%.

Erfindungsgemäß insbesondere bevorzugt ist es dabei, wenn die kompatiblen Solute ausgewählt sind aus Di-myo-inositol-phosphat (DIP), cyclisches 2,3-diphosphoglycerat (cDPG), 1,1- Diglycerin-Phosphat (DGP), ß-Mannosylglycerat (Firoin), ß- Mannosylglyceramid (Firoin-A) oder/und Dimannosyl-di-inositolphosphat (DMIP), Ectoin, Hydroxyectoin oder Mischungen davon.

Unter den ebenfalls bevorzugt eingesetzten Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Zubereitungen, die ein Aryloxim, vorzugsweise 2-Hydroxy-5-methyllaurophenonoxim enthalten, zeigen überraschende antiinflammatorische Eignung. Dabei enthalten die Zubereitungen vorzugsweise 0,01 bis 10 Gew.-% des Aryloxims, wobei es insbesondere bevorzugt ist, wenn die Zubereitung 0,05 bis 5 Gew.-% Aryloxim enthält.

In einer weiteren ebenfalls bevorzugten Ausführungsform der vorliegenden Erfindung enthält die erfindungsgemäße Zubereitung mindestens einen Selbstbräuner.

Als vorteilhafte Selbstbräuner können unter anderem eingesetzt werden:

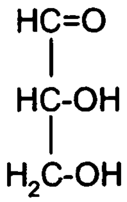

Glycerolaldehyd

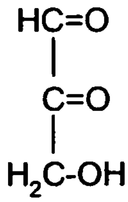

Hydroxymethylglyoxal

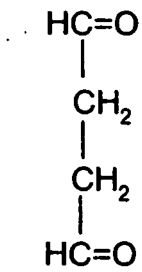

γ-Dialdehyd

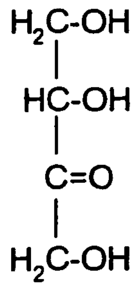

Erythrulose

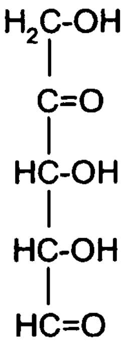

6-Aldo-D-Fructose

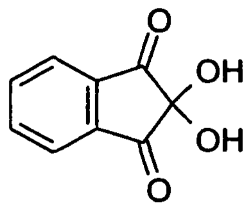

Ninhydrin.

Ferner ist das 5-Hydroxy-1,4-naphtochinon (Juglon) zu nennen, das aus den Schalen frischer Walnüsse extrahiert werden kann sowie das in den Henna-Blättern vorkommende 2-Hydroxy-1,4-naphtochinon (Lawson). Auch das Flavonoid Diosmetin und seine Glycoside oder Sulfate können eingesetzt werden. Dabei können diese Verbindungen in Form von Reinstoffen oder Pflanzenextrakten eingesetzt werden. Diosmetin kann beispielsweise vorzzugsweise in Form eines Chrysanthemum Extraktes eingesetzt werden.

Ganz besonders bevorzugt ist das 1,3-Dihydroxyaceton (DHA), ein im menschlichen Körper vorkommender dreiwertiger Zucker und dessen Derivate.

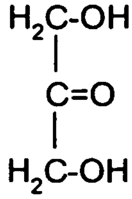

1,3-Dihydroxyaceton (DHA)

Dabei können die genannten Selbstbräuner alleine oder als Gemisch eingesetzt werden. Insbesondere bevorzugt ist es dabei, wenn DHA im Gemisch mit einem weiteren der oben genannten Selbstbräuner eingesetzt wird.

Ferner können die erfindungsgemäßen Zubereitungen auch Farbstoffe und Farbpigmente enthalten. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. $Fe_2O_3$, $Fe_3O_4$, FeO(OH)) und/oder Zinnoxid. Vorteilhafte Farbstoffe sind beispielsweise Carmin, Berliner Blau, Chromoxidgrün, Ultramerinblau und/oder Manganviolett. Es ist insbesondere vorteilhaft, die Farbstoffe und/oder Farbpigmente aus der folgenden Liste zu wählen. Die Colour Index Nummern (CIN) sind dem Rowe Colour Index, 3. Auflage, Society of Dyers and Colourists, Bradford, England, 1971 entnommen.

| Chemische oder sonstige Bezeichnung | CIN | Farbe |
|---|---|---|
| Pigment Green | 10006 | grün |
| Acid Green 1 | 10020 | Grün |
| 2,4-Dinitrohydroxynaphthalin-7-sulfonsäure | 10316 | Gelb |
| Pigment Yellow 1 | 11680 | Gelb |
| Pigment Yellow 3 | 11710 | Gelb |
| Pigment Orange 1 | 11725 | Orange |
| 2,4-Dihydroxyazobenzol | 11920 | Orange |
| Solvent Red 3 | 12010 | Rot |
| 1-(2'-Chlor-4'-nitro-1'-phenylazo)-2-hydroxynaphthalin | 12085 | Rot |
| Pigment Red 3 | 12120 | Rot |
| Ceresrot; Sudanrot; Fettrot G | 12150 | Rot |
| Pigment Red 112 | 12370 | Rot |
| Pigment Red 7 | 12420 | Rot |
| Pigment Brown 1 | 12480 | Braun |
| 4-(2'-Methoxy-5'sulfonsäurediethylamid-1'-phenylazo)-3-hydroxy-5"-chloro-2",4"-dimethoxy2-naphthoesäureanilid | 12490 | Rot |
| Disperse Yellow 16 | 12700 | Gelb |
| 1-(4-Sulfo-1-phenylazo)-4-amino-benzol-5-sulfosäure | 13015 | Gelb |
| 2,4-Dihydroxy-azobenzol-4'-sulfosäure | 14270 | Orange |
| 2-(2,4-Dimethylphenylazo-5-sulfosäure)-1-hydroxynaphthalin-4-sulfosäure | 14700 | Rot |
| 2-(4-Sulfo-1-naphthylazo)-1-naphthol-4-sulfosäure | 14720 | Rot |
| 2-(6-Sulfo-2,4-xylylazo)-1-naphthol-5-sulfosäure | 14815 | Rot |
| 1-(4'-Sulfophenylazo)-2-hydroxynaphthalin | 15510 | Orange |
| 1-(2-Sulfosäure-4-chlor-5-carbonsäure-1-phenylazo)-2-hydroxynaphthalin | 15525 | Rot |
| 1-(3-Methyl-phenylazo-4-sulfosäure)-2-hydroxynaphthalin | 15580 | Rot |
| 1-(4',(8')-Sulfosäurenaphthylazo)-2-hydroxynaphthalin | 15620 | Rot |
| 2-Hydroxy-1,2'-azonaphthalin-1'-sulfosäure | 15630 | Rot |
| 3-Hydroxy-4-phenylazo-2-naphthylcarbonsäure | 15800 | Rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxynaphthalin-3-carbonsäure | 15865 | Rot |
| 1-(2-Sulfo-1-naphthylazo)-2-hydroxynaphthalin-3-carbonsäure | 15880 | Rot |
| 1-(3-Sulfo-1-phenylazo)-2-naphthol-6-sulfosäure | 15980 | Orange |
| 1-(4-Sulfo-1-phenylazo)-2-naphthol-6-sulfosäure | 15985 | Gelb |
| Allura Red | 16035 | Rot |
| 1-(4-Sulfo-1-naphthylazo)-2-naphthol-3,6-disulfosäure | 16185 | Rot |
| Acid Orange 10 | 16230 | Orange |
| 1-(4-Sulfo-1-naphthylazo)-2-naphthol-6,8-disulfosäure | 16255 | Rot |
| 1-(4-Sulfo-1-naphthylazo)-2-naphthol-3,6,8-trisulfosäure | 16290 | Rot |
| 8-Amino-2-phenylazo-1-naphthol-3,6-disulfosäure | 17200 | Rot |
| Acid Red 1 | 18050 | Rot |

(fortgesetzt)

| Chemische oder sonstige Bezeichnung | CIN | Farbe |
|---|---|---|
| Acid Red 155 | 18130 | Rot |
| Acid Yellow 121 | 18690 | Gelb |
| Acid Red 180 | 18736 | Rot |
| Acid Yellow 11 | 18820 | Gelb |
| Acid Yellow 17 | 18965 | Gelb |
| 4-(4-Sulfo-1-phenylazo)-1-(4-sulfophenyl)-5-hydroxypyrazolon-3-carbonsäure | 19140 | Gelb |
| Pigment Yellow 16 | 20040 | Gelb |
| 2,6-(4'-Sulfo-2",4"-dimethyl)-bis-phenylazo)1,3-dihydroxybenzol | 20170 | Orange |
| Acid Black 1 | 20470 | schwarz |
| Pigment Yellow 13 | 21100 | Gelb |
| Pigment Yellow 83 | 21108 | Gelb |
| Solvent Yellow | 21230 | Gelb |
| Acid Red 163 | 24790 | Rot |
| Acid Red 73 | 27290 | Rot |
| 2-[4'-(4"-Sulfo-1"-phenylazo)-7'-sulfo-1'-naphthylazo]-1-hydroxy-7-aminonaphthalin-3,6-disulfosäure | 27755 | schwarz |
| 4-[4"-Sulfo-1 "-phenylazo)-7'-sulfo-1'-naphthylazo]-1-hydroxy-8-acetyl-aminonaphthalin-3,5-disulfosäure | 28440 | schwarz |
| Direct Orange 34, 39, 44, 46, 60 | 40215 | Orange |
| Food Yellow | 40800 | Orange |
| trans-ß-Apo-8'-Carotinaldehyd ($C_{30}$) | 40820 | Orange |
| trans-Apo-8'-Carotinsäure ($C_{30}$)-ethylester | 40850 | Orange |
| Canthaxanthin | 40850 | Orange |
| Acid Blue 1 | 42045 | Blau |
| 2,4-Disulfo-5-hydroxy-4'-4"-bis-(diethylamino)triphenylcarbinol | 42051 | Blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | Grün |
| Acid Blue 7 | 42080 | Blau |
| (N-Ethyl-p-sulfobenzyl-amino)-phenyl-(2-sulfophenyl)-methylen-(N-ethyl-N-p-sulfo-benzyl) $\Delta^{2,5}$-cyclohexadienimin | 42090 | Blau |
| Acid Green 9 | 42100 | Grün |
| Diethyl-di-sulfobenzyl-di-4-amino-2-chlor-di-2-methyl-fuchsonimmonium | 42170 | Grün |
| Basic Violet 14 | 42510 | Violett |
| Basic Violet 2 | 42520 | Violett |
| 2'-Methyl-4'-(N-ethyl-N-m-sulfobenzyl)-amino-4"-(N-diethyl)-amino-2-methyl-N-ethylIN-m-sulfobenzylfuchsonimmonium | 42735 | Blau |
| 4'-(N-Dimethyl)-amino-4"-(N-phenyl)-aminonaphtho-N-dimethylfuchsonimmonium | 44045 | Blau |
| 2-Hydroxy-3,6-disulfo-4,4'-bis-dimethylaminonaphthofuchsonimmonium | 44090 | Grün |
| Acid Red 52 | 45100 | Rot |
| 3-(2'-Methylphenylamino)-6-(2'-methyl-4'-sulfophenylamino)-9-(2"-carboxyphenyl)-xantheniumsalz | 45190 | Violett |
| Acid Red 50 | 45220 | Rot |
| Phenyl-2-oxyfluoron-2-carbonsäure | 45350 | gelb |
| 4,5-Dibromfluorescein | 45370 | Orange |
| 2,4,5,7-Tetrabromfluorescein | 45380 | Rot |
| Solvent Dye | 45396 | Orange |
| Acid Red 98 | 45405 | Rot |
| 3',4',5',6'-Tetrachlor-2,4,5,7-tetrabromfluorescein | 45410 | Rot |
| 4,5-Diiodfluorescein | 45425 | Rot |

(fortgesetzt)

| Chemische oder sonstige Bezeichnung | CIN | Farbe |
|---|---|---|
| 2,4,5,7-Tetraiodfluorescein | 45430 | Rot |
| Chinophthalon | 47000 | Gelb |
| Chinophthalon-disulfosäure | 47005 | Gelb |
| Acid Violet 50 | 50325 | Violett |
| Acid Black 2 | 50420 | Schwarz |
| Pigment Violet 23 | 51319 | Violett |
| 1,2-Dioxyanthrachinon, Calcium-Sluminiumkomplex | 58000 | Rot |
| 3-Oxypyren-5,8,10-sulfosäure | 59040 | Grün |
| 1-Hydroxy-4-N-phenyl-aminoanthrachinon | 60724 | Violett |
| 1-Hydroxy-4-(4'-methylphenylamino)-anthrachinon | 60725 | Violett |
| Acid Violet 23 | 60730 | Violett |
| 1,4-Di(4'-methyl-phenylamino)-anthrachinon | 61565 | Grün |
| 1,4-Bis-(o-sulfo-p-toluidino)-anthrachinon | 61570 | Grün |
| Acid Blue 80 | 61585 | Blau |
| Acid Blue 62 | 62045 | Blau |
| N,N'-Dihydro-1,2,1',2'-anthrachinonazin | 69800 | Blau |
| Vat Blue 6; Pigment Blue 64 | 69825 | Blau |
| Vat Orange 7 | 71105 | orange |
| Indigo | 73000 | Blau |
| Indigo-disulfosäure | 73015 | Blau |
| 4,4'-Dimethyl-6,6'-dichlorthioindigo | 73360 | Rot |
| 5,5'Dichlor-7,7'-dimethylthioindigo | 73385 | violett |
| Quinacridone Violet 19 | 73900 | violett |
| Pigment Red 122 | 73915 | Rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6, 19; Natural Red 1 | 75100 | gelb |
| Bixin, Nor-Bixin | 75120 | orange |
| Lycopin | 75125 | gelb |
| trans-alpha-, bet- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | Rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | Rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268 :1 | schwarz |

(fortgesetzt)

| Chemische oder sonstige Bezeichnung | CIN | Farbe |
|---|---|---|
| Chromoxid | 77288 | grün |
| Chromoxid, wasserhaltig | 77278 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyahoferrat | 77510 | blau |
| Pigment White 18 | 77713 | weiß |
| Mangananimoniumdiphosphat | 77742 | violett |
| Manganphosphat; $Mn_3(PO_4)_2 \cdot 7\ H_2O$ | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalzem, Anthocyane | | rot |
| Aluminium-, Zink-, Magnesium- und Calciumstearat | | weiß |
| Bromthymolblau | | blau |

Es kann ferner günstig sein, als Farbstoff eine oder mehrerer Substanzen aus der folgenden Gruppe zu wählen: 2,4-Dihydroxyazobenzol, 1-(2'-Chlor-4'-nitro-1'phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfonsäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfonsäure, Aluminiumsalz der 4-(4-Sulfo-1-phenylazo)-2-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77492), Manganammoniumdiphosphat und Titandioxid.

Ferner vorteilhaft sind öllösliche Naturfarbstoffe, wie z.B. Paprikaextrakt, β-Carotin oder Cochenille.

Vorteilhaft im Sinne der vorliegenden Erfindung sind ferner Gelcrèmes mit einem Gehalt an Perlglanzpigmenten. Bevorzugt sind insbesondere die im folgenden aufgelisteten Arten von Perlglanzpigmenten:

1. Natürliche Perlglanzpigmente, wie z.B.

- "Fischsilber" (Guanin/Hypoxanthin-Mischkristalle aus Fischschuppen) und
- "Perlmutt" (vermahlene Muschelschalen)

2. Monokristalline Perlglanzpigmente wie z.B. Bismuthoxychlorid (BiOCl)
3. Schicht-Substrat Pigmente: z.B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteilhaft ist z.B. das unter der CIN 77163 aufgelistete Glanzpigment.

Vorteilhaft sind ferner beispielsweise die folgenden Perlglanzpigmentarten auf Basis von Glimmer/Metalloxid:

| Gruppe | Belegung/Schichtdicke | Farbe |
|---|---|---|
| **Silberweiße Perlglanzpigmente** | $TiO_2$: 40-60 nm | silber |
| **Interferenzpigmente** | $TiO_2$: 60-80 nm | gelb |
| | $TiO_2$: 80-100 nm | rot |
| | $TiO_2$: 100-140 nm | blau |
| | $TiO_2$: 120-160 nm | grün |
| **Farbglanzpigmente** | $Fe_2O_3$ | bronze |
| | $Fe_2O_3$ | kupfer |
| | $Fe_2O_3$ | rot |
| | $Fe_2O_3$ | rotviolett |
| | $Fe_2O_3$ | rotgrün |
| | $Fe_2O_3$ | schwarz |
| **Kombinationspigmente** | $TiO_2$ / $Fe_2O_3$ | Goldtöne |
| | $TiO_2$ / $Cr_2O_3$ | grün |
| | $TiO_2$ / Berliner Blau | tiefblau |

Besonders bevorzugt sind z.B. die von der Firma Merck KGaA, Darmstadt unter den Handelsnamen Timiron®, Colorona® oder Dichrona® erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z.B. Silica und dergleichen mehr. Vorteilhaft sind z.B. mit $TiO_2$ und $Fe_2O_3$ beschichtete $SiO_2$-Partikel ("Ronaspheren"), die von der Firma Merck KGaA, Darmstadt vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Perlglanzpigmente, welche unter der Verwendung von $SiO_2$ hergestellt werden. Solche Pigmente , die auch zusätzlich gonichromatische Effekte haben können, sind z.B. unter dem Handelsnamen Sicopearl® Fantastico bei der Firma BASF AG, Ludwigshafen, erhältlich.

Weiterhin vorteilhaft können Pigmente der Firma Engelhard / Mearl auf Basis von Calcium Natrium Borosilikat, die mit Titandioxid beschichtet sind, eingesetzt werden. Diese sind unter dem Namen Reflecks® erhältlich. Sie weisen durch ihre Partikelgröße von 40-80 $\mu$m zusätzlich zu der Farbe einen Glitzereffekt auf.

Besonders vorteilhaft sind ferner auch Effektpigmente, welche unter der Handelsbezeichnung Metasomes® Standard/Glitter in verschiedenen Farben (yellow, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.

Alle Verbindungen oder Komponenten, die in den Zubereitungen verwendet werden können, sind entweder bekannt und käuflich erwerbbar oder können nach bekannten Verfahren synthetisiert werden. Der Zubereitung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

In einer bevorzugten Ausführungsform enthalten die erfindungsgemäßen Zubereitungen hydrophile Tenside.

Die hydrophilen Tenside werden bevorzugt gewählt aus der Gruppe der Alkylglucoside, der Acyllactylate, der

Betaine sowie der Cocoamphoacetate. Die Alkylglucoside werden ihrerseits vorteilhaft gewählt aus der Gruppe der Alkylglucoside, welche sich durch die Strukturformel

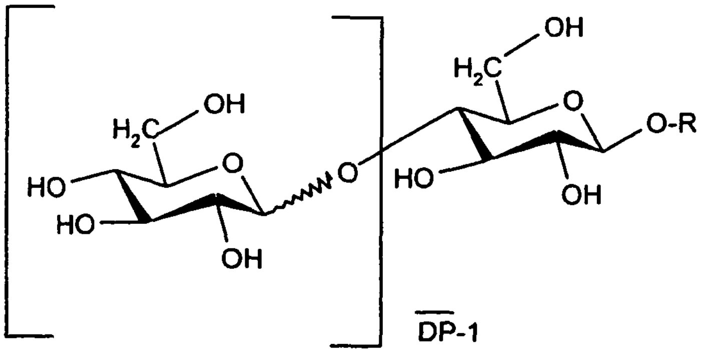

auszeichnen, wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet. Der Wert *DP* repräsentiert den Glucosidierungsgrad der erfindungsgemäß verwendeten Alkylglucoside und ist definiert als

$$\overline{DP} = \frac{p_1}{100} \cdot 1 + \frac{p_2}{100} \cdot 2 + \frac{p_3}{100} \cdot 3 + \ldots = \sum \frac{p_i}{100} \cdot i$$

Dabei stellen $p_1$, $p_2$, $p_3$ ... bzw. $p_i$ den Anteil der einfach, zweifach dreifach ... i-fach glucosylierten Produkte in Gewichtsprozenten dar. Erfindungsemäß vorteilhaft werden Produkte mit Glucosylierungsgraden von 1-2, insbesondere vorteilhaft von 1, 1 bis 1,5, ganz besonders vorteilhaft von 1,2-1,4, insbesondere von 1,3 gewählt.

Der Wert DP trägt dem Umstande Rechnung, dass Alkylglucoside herstellungsedingt in der Regel Gemische aus Mono- und Oligoglucosiden darstellen. Erfindungsgemäß vorteilhaft ist ein relativ hoher Gehalt an Monoglucosiden, typischerweise in der Größenordnung von 40-70 Gew.-%.

Besonders vorteilhaft verwendete Alkylglylcoside werden gewählt aus der Gruppe Octylglucopyranosid, Nonylglucopyranosid, Decylglucopyranosid, Undecylglucopyranosid, Dodecylglucopyranosid, Tetradecylglucopyranosid und Hexadecylglucopyranosid.

Es ist ebenfalls von Vorteil, natürliche oder synthetische Roh- und Hilfsstoffe bzw. Gemische einzusetzen, welche sich durch einen wirksamen Gehalt an den verwendeten Wirkstoffen auszeichnen, beispielsweise Plantaren® 1200 (Henkel KGaA), Oramix® NS 10 (Seppic).

Die Acyllactylate werden ihrerseits vorteilhaft gewählt aus der Gruppe der Substanzen, welche sich durch die Strukturformel

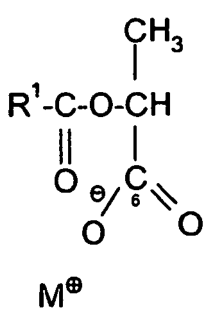

auszeichnen, wobei $R^1$ einen verzweigten oder unverzweigten Alkylrest mit 1 bis 30 Kohlenstoffatomen bedeutet und $M^+$ aus der Gruppe der Alkaliionen sowie der Gruppe der mit einer oder mehreren Alkyl- und/oder mit einer oder mehreren Hydroxyalkylresten substituierten Ammoniumionen gewählt wird bzw. dem halben Äquivalent eines Erdalkalions entspricht.

Vorteilhaft ist beispielsweise Natriumisostearyllactylat, beispielsweise das Produkt Pathionic® ISL von der Gesellschaft American Ingredients Company.

Die Betaine werden vorteilhaft gewählt aus der Gruppe der Substanzen, welche sich durch die Strukturformel

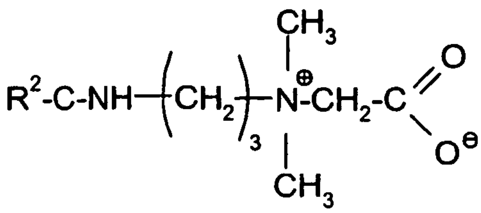

auszeichnen, wobei $R^2$ einen verzweigten oder unverzeigten Alkylrest mit 1 bis 30 Kohlenstoffatomen bedeutet.

Insbesondere vorteilhaft bedeutet $R^2$ einen verzweigten oder unverzweigten Alkylrest mit 6 bis 12 Kohlenstoffatomen.

Vorteilhaft ist beispielsweise Capramidopropylbetain, beispielsweise das Produkt Tego® Betain 810 von der Gesellschaft Th. Goldschmidt AG.

Als erfindungsgemäß vorteilhaftes Cocoamphoacetat wird beispielsweise Natriumcocoamphoacetat gewählt, wie es unter der Bezeichnung Miranol® Ultra C32 von der Gesellschaft Miranol Chemical Corp. erhältlich ist.

Die erfindungsgemäßen Zubereitungen sind vorteilhaft dadurch gekennzeichnet, dass das oder die hydrophilen Tenside in Konzentrationen von 0,01-20 Gew.-% bevorzugt 0,05-10 Gew.-%, besonders bevorzugt 0,1-5 Gew.-%, jeweils bezogen auf das Gesamtgewicht der Zusammensetzung, vorliegt oder vorliegen.

Zu Anwendung werden die erfindungsgemäßen kosmetischen und dermatologischen Zubereitungen in der für Kosmetika üblichen Weise auf die Haut und/oder die Haare in ausreichender Menge aufgebracht.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -Milch vorliegt und beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält. Eine bevorzugte Ausführungsform sind auch Hydrogele.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen fotochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shamponieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung mit Lichtschutzeigenschaften kann verschiedene, in diesem Mitteltyp verwendete Adjuvantien enthalten, wie Grenzflächen aktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Die erfindungsgemäßen Zubereitungen können dabei mit Hilfe von Techniken hergestellt werden, die dem Fachmann wohl bekannt sind. Die erfindungsgemäßen Substanzen lassen sich dabei direkt ohne weitere vorbereitende Maßnahmen in kosmetischen Zubereitungen einarbeiten.

Vorteilhaft können die Zubereitungen erfindungsgemäß, wie vorstehend schon beschrieben, weitere UV-Filtersubstanzen enthalten, wobei die Gesamtmenge der Filtersubstanzen z.B. 0,1 bis 30 Gew.-%, vorzugsweise 0,5 bis 10 Gew.-%, insbesondere 1 bis 6 Gew.-% beträgt, bezogen auf das Gesamtgewicht der Zubereitungen.

Weiterhin können die erfindungsgemäßen Zubereitungen auch als pharmazeutische Mittel zur vorbeugenden Behandlung von Entzündungen und Allergien der Haut sowie auch in bestimmten Fällen zur Verhütung bestimmter Krebsarten verwendet werden. Das erfindungsgemäße pharmazeutische Mittel kann oral oder topisch verabreicht werden.

Die erfindungsgemäßen Zubereitungen können mit Hilfe von Techniken hergestellt werden, die dem Fachmann wohl bekannt sind. Auch ohne weitere Ausführungen wird davon ausgegangen, dass ein Fachmann die obige Beschreibung in weitesten Umfang nutzen kann. Die bevorzugten Ausführungsformen sind deswegen lediglich als beschreibende,

keineswegs als in irgendeine Weise limitierende Offenbarung aufzufassen. Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. das Gesamtgewicht der Zubereitungen bezogen. Die vollständige Offenbarung aller vor- und nachstehend aufgeführten Anmeldungen und Veröffentlichungen sind durch Bezugnahme in diese Anmeldung eingeführt. In diesen Beispielen wird als bevorzugte Verbindung gemäß Formel I das N,N-Dimethyldecanamid Spectrasolv DMDA (Jiangsu Feixang Chemicals oder Lehmann & Voss) verwendet. Der bevorzugte schwerlösliche organische UV-Filter ist Ethylhexyl Triazone.

**Beispiele:**

Allgemein: Bestimmung der Kapselgröße

Die Partikelgrössenbestimmung erfolgt mittels Laserbeugung nach ISO/DIS 13320, unter den Bedingungen, wie folgt:

Gerät: Mastersizer 2000 der Fa. Malvern mit der Dispergiereinheit Hydro 2000G

Die Einwaage erfolgt als Direktzugabe mit Einmalpipette.
Dispergiermedium: Wasser
Probenvorbereitung: Keine
Dispergierhilfe: Keine
Rührerdrehzahl: 800 upm
Pumpendrehzahl: 1800 upm
Ultraschall: Ohne
Brechungsindex Partikel: 1,55
Absorption: 0,001
Brechungsindex Dispergiermedium: 1,33
Messzeit [sec/Snaps]: 5/5000
Hintergrundmesszeit [sec/Snaps]: 8/8000
Anzahl Messungen: 1
Obscuration [%]: 8-12

Der in vivo SPF (sun protection factor) wurde nach der internationalen Methode gemessen, wie publiziert in COLIPA 001-2003 - siehe ausführlich Beispiel 6. [COLIPA the European cosmetic toiletry and perfumery association]

Der in vitro (UV)APF wurde nach der Deutschen Norm DIN 67502, "Charakterisierung der UVA-Schutzwirkung von dermalen Sonnenschutzmitteln durch Transmissionsmessungen unter Berücksichtigung des Lichtschutzfaktors", 3. Norm-Vorlage, März 2003 bestimmt.

Beispiel 1:

Eine Lösung aus 400 g Ethylhexyl Triazone, 400 g Dimethyl Capramide (Spectrasolv DMDA) und 240 g Tetraethylorthosilikat wird unter Kühlung mit Hilfe eines Emulgierwerkzeuges (Ultra Turax) in einer Tensidlösung [448g VE-Wasser und 11 g Cetyltrimethyammoniumchlorid (CTAC)] emulgiert. Die fertige Emulsion wird unter Rühren in salzsaures Wasser gegeben. Die erhaltene Mischung wird 48 - 72 h bei Raumtemperatur gerührt. Danach wird das bei der Hydrolyse des Alkylsilanes entstandene Ethanol destillativ abgereichert. Nach Zugabe von 20 g PVP in 300 g VE-Wasser wird mit Na-Citrat-Lösung der pH-Wert des Rückstandes auf 3,4 - 3,6 eingestellt und mit VE-Wasser aufgefüllt.

Der Wirkstoffgehalt der Suspension beträgt 18 Gew.-%.

Die Einarbeitung in die kosmetische Zubereitung kann in dieser Form erfolgen.

Die Isolierung der Silica-Kapsel ist nach herkömmlichen Methoden möglich.

Partikelgröße: d(0,50) = 0,53μm, d(0,90) = 1,39μm

Beispiel 2:

Im Vergleich zu Beispiel 1 wurden UV-Filter-Kapseln mit im Markt bekannten Lösungsmitteln hergestellt.

Die Löslichkeit von Ethylhexyl Triazone in den angegebenen Systemen lässt sich wie folgt bestimmen:

Die Testsubstanz wird in kleinen Bechergläsern auf einem beheizbaren Magnetrührer in das Lösungsmittel (z.B. kosmetisches Emollient, wie in der Tabelle angegeben) unter Rühren zunächst bei Raumtemperatur (ca. 20°C - 25°C) eingearbeitet (die Rührzeit sollte dabei ca. 30 min. nicht überschreiten). Die Beurteilung erfolgt visuell, d.h. es wird überprüft, ob eine vollständig klare Lösung und ohne erkennbare Partikel vorliegt. Eingesetzte Mengen, Konditionen

und Rührzeiten sind zu notieren. Ist die Substanz gut löslich, wird die Konzentration durch nachträgliches Einarbeiten weiterer Substanz erhöht, z.B. durch Zugabe unter Rühren in 0,1 g Schritten. Die Ansatzgrösse beträgt üblicherweise 10 g, d.h. für eine Löslichkeit von 1% (w/w): 0,1 g Testsubstanz in 9,9 g Lösungsmittel. Die Beurteilung erfolgt visuell, evtl. unter Zuhilfenahme eines Mikroskops mit Lamda-Plättchen.

Gehaltsbestimmung der UV-Filter: HPLC, Bestimmung mit externem Standard. Chromatographisches System: Stainless steel cartridge LiChroCART® 250 mm 4 mm, Superspher® 100 RP-18 endcapped, Korngröße 4 $\mu$m. Säulentemperatur: Raumtemperatur
Mobile Phase: Methanol / Lösung A (80:20); Flußrate 1.0 ml/min. Detektion: UV-Detektor.

Probenvorbereitung:

Probenlösung: Ca. 30 mg des Produkts, genau gewogen, wird in einem 100 ml Messkolben mit 40 ml Methanol dispergiert. Diese Mischung wird 5 Minuten im Ultraschallbad gemischt. Nach dem Abkühlen wird mit Methanol bis zur Marke aufgefüllt. Entsprechend wird zur Messung verdünnt. Vor der Injektion wird die Mischung mit einem Spritzenfilter Anotop® 25 (pore size 0.02 $\mu$m) filtriert.

Vergleichslösung: Ca 30 mg Ethylhexyl Triazone, genau gewogen, werden in einem 100 ml Messkolben in Methanol gelöst und es wird bis zur Marke aufgefüllt. 1,0 ml dieser Lösung wird in einem 50 ml Messkolben mit Methanol auf 50 ml verdünnt.

Die Auswertung erfolgt zum Beispiel mit Agilent HPLC ChemStation; d.h. es erfolgt eine Auswertung der Peakflächen nach der Methode externer Standardauswertung.

Löslichkeiten von Ethylhexyl Triazone bei Raumtemperatur (20° bis 25°C) in verschiedenen Lösungsmitteln und Gehalt in der wässrigen Dispersion

| Lösungsmittel | Ethylhexyl Triazone Löslichkeit bei 25°C | Gehalt* |
|---|---|---|
| Dioctyl Malate | 13% | 4,4% |
| C12-13 Alkyl Lactate | 22% | 7,5% |
| Di-C12-13 Alkyl Tartrate | 35% | 11.9% |
| Spectrasolv DMDA | Ca. 55% | 18,7% |
| *bei 34% Kapselinhalt und 60% $H_2O$ | | |

Die mit dem erfindungsgemäßen Emollient erzielbaren Gehälter von maximal 18.7% bei 34% Kapselinhalt und 60% Wassergehalt ist deutlich höher, ca. 57% höher, als beim bisherigen Stand der Technik.

Beispiel 3:

Eine Lösung aus 240 g Bis-Ethylhexyloxyphenol Methoxyphenyl Triazine, 560 g Dimethyl Capramide (Spectrasolv DMDA) und 240 g Tetraethylorthosilikat wird unter Kühlung mit Hilfe eines Emulgierwerkzeuges (Ultra Turax) in einer Tensidlösung [448g VE-Wasser und 11 g Cetyltrimethyammoniumchlorid (CTAC)] emulgiert. Die fertige Emulsion wird unter Rühren in salzsaures Wasser gegeben. Die erhaltene Mischung wird 48 - 72 h bei Raumtemperatur gerührt. Danach wird das bei der Hydrolyse des Alkylsilanes entstandene Ethanol destillativ abgereichert. Nach Zugabe von 20 g PVP in 300 g VE-Wasser wird mit Na-Citrat-Lösung der pH-Wert des Rückstandes auf 3,4 - 3,6 eingestellt und mit VE-Wasser aufgefüllt.

Der Wirkstoffgehalt der Suspension beträgt 10,8 Gew.-%.

Die Einarbeitung in die kosmetische Zubereitung kann in dieser Form erfolgen.

Die Isolierung der Silica-Kapsel ist nach herkömmlichen Methoden möglich.

Partikelgröße: d(0,50) = 0,53$\mu$m, d(0,90) = 1,39$\mu$m

<u>Beispiel 4:</u> Formulierungsbeispiele:

**A) Hydrogel**

| Rohstoff | INCI | 43-06-H-2 [%] | [g] |
|---|---|---|---|
| **UV-Filter-Kapsel** (17,5 % Ethylhexyl Triazone in Spectrasolv DMDA) | | **10,00** | **20,00** |
| Lubrajel DV | PROPYLENE GLYCOL, POLYGLYCERYLMETHACRYLATE | 20,00 | 40,00 |
| RonaCare® Ectoin | ECTOIN | 0,50 | 1,00 |
| Wasser, demineralisiert | AQUA (WATER) | 68,80 | 137,60 |
| Germaben II | PROPYLENE GLYCOL, DIAZOLIDINYL UREA, METHYLPARABEN, PROPYLPARABEN | 0,70 | 1,40 |
| | | **100,00** | **200,00** |

**Herstellung:**

Lubrajel vorlegen und unter Rühren übrige Bestandteile hinzufügen. In vivo SPF: 4.6; UVA-PF: 1

**B) Öl-in-Wasser**

| 0,1 % Neolone 950 Rohstoff | INCI | 43-06-OW-2 [%] | [g] |
|---|---|---|---|
| **A** | | | |
| Eusolex® 9020 | BUTYL, METHOXYDIBENZOYL-METHANE | 2,00 | 5,00 |
| Paraffin dickflüssig | PARAFFINUM LIQUIDUM (MINERAL OIL) | 4,50 | 11,25 |
| Pelemol BIP | ISOPROPYLPHTALIMIDE, BUTYLPHTALIDE | 6,00 | 15,00 |
| Isopropylpalmitat | ISOPROPYL PALMITATE | 7,50 | 18, 75 |
| Sojaöl | GLYCINE SOJA (SOYBEAN OIL) | 5,00 | 12,50 |
| RonaCare® Tocopherolacetat | TOCOPHERYL ACETATE | 1,00 | 2,50 |
| Carbopol Ultrez 10 | CARBOMER | 0,30 | 0,75 |
| **B** | | | |
| **UV-Filter-Kapsel** (17,5 % Ethylhexyl Triazone in Spectrasolv DMDA) | | **27,00** | **67,50** |
| Wasser, demineralisiert | AQUA (WATER) | 38,50 | 96,25 |
| Sisterna L70-C | AQUA (WATER), SUCROSE LAURATE, ALCOHOL | 6,00 | 15,00 |
| Phenonip | PHENOXYETHANOL, BUTYLPARABEN, ETHYLPARABEN, PROPYLPARABEN; METHYLPARABEN | 1,00 | 2,50 |
| **C** | | | |
| Natronlauge, 10%ig | SODIUM HYDROXIDE | 1,20 | 3,00 |
| | | **100,00** | **250,00** |

**Herstellung**

Phase A bis auf Carbopol zusammengeben und Eusolex® 9020 verlösen. Falls nötig, auf ca. 50 C erwärmen. Carbopol einarbeiten und vorgelöste Phase B unter Rühren einemulgieren. Homogenisieren. Nach Zugabe von Phase C nochmals kurz homogenisieren.

In vivo SPF: 14,3; UVA-PF: 4,5

**C) Wasser-in-Öl**

| **Inhaltstoff** | **INCI** | **43-06-WO-2 [%]** | **[g]** |
|---|---|---|---|
| **A** | | | |
| Eusolex® T-2000 | TITANIUM DIOXIDE, ALUMINA, SIMETHICONE | 10,00 | 25,00 |
| Arlacel P135 | PEG-30 DIPOLYHYDROXYSTEARATE | 2,50 | 6,25 |
| Abil WE 09 | POLYGLYCERYL-4 ISOSTEARATE, CETYL PEG/PPG-10/1 DIME | 2,50 | 6,25 |
| Cetiol A | HEXYL LAURATE | 10,00 | 25,00 |
| Cetiol 868 | ETHYLHEXYL STEARATE | 14,00 | 35,00 |
| Shea butter | BUTYROSPERMUM PARKII (SHEA BUTTER) | 1,00 | 2,50 |
| Paracera M | MICROWAX | 0,50 | 1,25 |
| Crodafos CES | CETEARYL, ALCOHOL, DICETYL PHOSPHATE, CETETH-10-PH | 1,00 | 2,50 |
| Dow Corning 200 (100cs) | DIMETHICONE | 1,00 | 2,50 |
| Dow Corning 345 | CYCLOMETHICONE | 1,00 | 2,50 |
| **B** | | | |
| **UV-Filter-Kapsel** (17,5 % Ethylhexyl Triazone in Spectrasolv DMDA) | | **18,90** | **47,25** |
| RonaCare® Ectoin | ECTOIN | 0,30 | 0,75 |
| Rona Care® Allantoin | ALLANTOIN | 0,20 | 0,50 |
| Propylenglykol, 1,2-Sodium chloride | SODIUM CHLORIDE | 0,40 | 1,00 |
| Titriplex® III | DISODIUM EDTA | 0,05 | 0,13 |
| Water, demineralized | AQUA (WATER) | 32,95 | 82,38 |
| **C** | | | |
| Phenonip | PHENOXYETHANOL, BUTYLPARABEN, ETHYLPARABEN, PRO | 0,70 | 1,75 |
| | | **100,00** | **250,00** |

__Herstellung__

Phase A wird kombiniert, ohne Eusolex® T-2000 und auf 80°C erhitzt. Danach wird Eusolex® T-2000 langsam zur heißen Ölphase unter Rühren zugegeben. Phase B wird hergestellt und auf 75°C erhitzt. Danach wird Phase B langsam unter Rühren zu Phase A zugegeben. Homegenisieren und Kühlen. Unter 35°C wird Phase C zugegeben.
In vivo SPF: 35,4; UVA-PF: 4,5

**D-1) Öl-in-Wasser**

| **Trade Name** | **INCI** | **Emulsion 1** mit 2 % Ethylhexyl Triazone [%] | **Emulsion 2** mit 4 % Ethylhexyl Triazone [%] |
|---|---|---|---|
| **A** | | | |
| Cetiol B | Dibutyl Adipate | 9,00 | 8,00 |
| Tegosoft TN | C12-15 Alkyl Benzoate | 9,00 | 8,00 |
| Myritol 331 | Cocoglycerides | 12,00 | 12,00 |

(fortgesetzt)

| Trade Name | INCI | Emulsion 1 mit 2 % Ethylhexyl Triazone [%] | Emulsion 2 mit 4 % Ethylhexyl Triazone [%] |
|---|---|---|---|
| **A** | | | |
| Eumulgin VL 75 | Lauryl Glucoside, Polyglyceryl-2-Dipolyhydroxystearate, Glycerin | 4,00 | 4,00 |
| Lanette O | Cetearyl Alcohol | 2,00 | 2,00 |
| **Uvinul T 150** | **Ethylhexyl Triazone** | | **2,00** |
| **B** | | | |
| Glycerin 87 % | Glycerin | 3,00 | 3,00 |
| Titriplex III | Disodium EDTA | 0,10 | 0,10 |
| Cremophor A 25 | Ceteareth-25 | 1,00 | 1,00 |
| Keltrol RD | Xanthan Gum | 0,30 | 0,30 |
| Veegum Ultra | Magnesium Aluminium Silicate | 1,50 | 1,50 |
| Wasser | Aqua (water) | ad 100,00 | ad 100,00 |
| **C** | | | |
| Citronensäure | Citric Acid | 0,50 | 0,50 |
| **UV-Filter-Kapsel** (16,6 % Ethylhexyl Triazone in Spectrasolv DMDA) entspricht 2 % Uvinul®T 150 | **Aqua (water), Ethylhexyl Triazone, Dimethyl Capramide, Silica, PVP, Chlorphenesin** | **12,10** | **12,10** |
| **Trade Name** | **INCI** | **Emulsion 1** mit 2 % Ethylhexyl Triazone [%] | **Emulsion 2** mit 4 % Ethylhexyl Triazone [%] |
| **D** | | | |
| Phenonip | Phenoxyethanol, Methylparaben, Ethylparaben, Butylparaben, Propylparaben, Isobutylparaben | 1,00 | 1,00 |

**Herstellung:**

Phase A zusammengeben und auf 80 °C erhitzen. Prüfen, ob der feste UV-Filter gelöst ist.

Phase B zusammengeben und ebenfalls auf 80 °C erhitzen. Phase B in Phase A unter Rühren einemulgieren und 3 min homogenisieren. Unter Rühren abkühlen, mit Citronensäure den pH-Wert einstellen und bei etwa 30 °C Eusolex® UV-Pearls™ Uvinul T 150 und Konservierungsmittel einarbeiten.

**D-2) Öl-in-Wasser**

| Rohstoff | INCI | [%] | [g] |
|---|---|---|---|
| **A** | | | |
| Tinosorb S | BIS-ETHYLHEXYLOXYPHENOL METHOXYPHENYL TRIAZINE | 2,00 | 5.00 |
| Spektrasolv DMDA | DIMETHYL CAPRAMIDE | 7,00 | 25,00 |
| Paraffin dickflüssig | PARAFFINUM LIQUIDUM (MINERAL OIL) | 3,00 | 5,00 |
| Pelemol BIP | ISOPROPYLPHTALIMIDE, BUTYLPHTALIDE | 6,00 | 15,00 |
| Sojaöl | GLYCINE SOJA (SOYBEAN OIL) | 5,00 | 12,50 |
| RonaCare® Tocopherolacetat | TOCOPHERYL ACETATE | 1,00 | 2,50 |
| Carbopol Ultrez 10 | CARBOMER | 0,30 | 0,75 |

(fortgesetzt)

| | | | |
|---|---|---|---|
| **B** | | | |
| **UV-Filter-Kapsel** (10,5 % Tinosorb S in Spectrasolv DMDA) | | **27,00** | **67,50** |
| Wasser, demineralisiert | AQUA (WATER) | ad100 | ad 250 |
| Sisterna L70-C | AQUA (WATER), SUCROSE LAURATE, ALCOHOL | 6,00 | 15,00 |
| Phenonip | PHENOXYETHANOL, BUTYLPARABEN, ETHYLPARABEN, PROPYLPARABEN; METHYLPARABEN | 1,00 | 2,50 |
| **C** | | | |
| Natronlauge, 10%ig | SODIUM HYDROXIDE | 1,20 | 3,00 |
| | | **100,00** | **250,00** |

**Herstellung**

Phase A bis auf Carbopol zusammengeben und Tinosorb S lösen. Falls nötig, auf ca. 60 C erwärmen. Carbopol einarbeiten und vorgelöste Phase B unter Rühren einemulgieren. Homogenisieren. Nach Zugabe von Phase C nochmals kurz homogenisieren.

In vivo SPF:10 ; UVA-PF:7,1

**E) Wasser-in-Öl**

| Inhaltstoff | INCI | [%] | [g] |
|---|---|---|---|
| **A** | | | |
| Tinosorb S | BIS-ETHYLHEXYLOXYPHENOL METHOXYPHENYL TRIAZINE | 2,00 | 5.00 |
| Spektrasolv DMDA | DIMETHYL CAPRAMIDE | 7,00 | 25,00 |
| Eusolex® T-2000 | TITANIUM DIOXIDE, ALUMINA, SIMETHICONE | 10,00 | 25,00 |
| Arlacel P135 | PEG-30 DIPOLYHYDROXYSTEARATE | 2,50 | 6,25 |
| Abil WE 09 | POLYGLYCERYL-4 ISOSTEARATE, CETYL PEG/PPG-10/1 DIME | 2,50 | 6,25 |
| Cetiol A | HEXYL LAURATE | 3,00 | 7,5 |
| Cetiol 868 | ETHYLHEXYL STEARATE | 14,00 | 35,00 |
| Shea butter | BUTYROSPERMUM PARKII (SHEA BUTTER) | 1,00 | 2,50 |
| Paracera M | MICROWAX | 0,50 | 1,25 |
| Crodafos CES | CETEARYL, ALCOHOL, DICETYL PHOSPHATE, CETETH-10-PH | 1,00 | 2,50 |
| Dow Corning 200 (100cs) | DIMETHICONE | 1,00 | 2,50 |
| Dow Corning 345 | CYCLOMETHICONE | 1,00 | 2,50 |
| **B** | | | |
| **UV-Filter-Kapsel** (10,5 % Tinosorb S in Spectrasolv DMDA) | | **18,90** | **47,25** |
| RonaCare® Ectoin | ECTOIN | 0,30 | 0,75 |
| Rona Care® Allantoin | ALLANTOIN | 0,20 | 0,50 |
| Propylenglykol, 1,2-Sodium chloride | SODIUM CHLORIDE | 0,40 | 1,00 |
| Titriplex® III | DISODIUM EDTA | 0,05 | 0,13 |
| Water, demineralized | AQUA (WATER) | ad 100 | ad 250 |

(fortgesetzt)

| | | | |
|---|---|---|---|
| **C** | | | |
| Phenonip | PHENOXYETHANOL, BUTYLPARABEN,ETHYLPARABE NE, PROPYLPARABEBE | 0,70 | 1,75 |
| | | **100,00** | **250,00** |

**Herstellung**

Phase A wird unter Rühren kombiniert, ohne Eusolex® T-2000 und auf 80°C erhitzt bis Tinosorb S gelöst ist. Danach wird Eusolex® T-2000 langsam zur heißen Ölphase unter Rühren zugegeben. Phase B wird hergestellt und auf 75°C erhitzt. Danach wird Phase B langsam unter Rühren zu Phase A zugegeben. Homegenisieren und kühlen. Unter 35°C wird Phase C zugegeben.

In vivo SPF:36,5 ; UVA-PF:12

**F) Wasser-in-Öl**

| Inhaltstoff | INCI | [%] | [g] |
|---|---|---|---|
| **A** | | | |
| Tinosorb S | BIS-ETHYLHEXYLOXYPHENOL METHOXYPHENYL TRIAZINE | 2,00 | 5.00 |
| Spektrasolv DMDA | DIMETHYL CAPRAMIDE | 7,00 | 25,00 |
| Eusolex® T-2000 | TITANIUM DIOXIDE, ALUMINA, SIMEETHICONE | 4,00 | 10,00 |
| RonaCare® AP | BIS-ETHYLHEXYL HYDROXYDIMETHOXY BENZYLMALONATE | 2,00 | 5,00 |
| Imwitor 372 P | GLYCERYL STEARATE CITRATE | 3,50 | 8,75 |
| Imwitor 380 | GLYCERYL COCOATE CITRATE | 2,00 | 5,00 |
| Weizenkeimöl raffiniert | TRITICUM VULGARE (WHEAT GERM OIL) | 2,00 | 5,00 |
| Propyl-4-hydroxybenzoat | PROPYLPARABEN | 0,05 | 0,13 |
| **B** | | | |
| **UV-Filter-Kapsel** (10,5 % Tinosorb S in Spectrasolv DMDA) | | **20,00** | **50,00** |
| Karion F flüssig | SORBITOL | 3,00 | 7,50 |
| Keltrol SF | XANTHAN GUM | 0,50 | 1,25 |
| Methyl-4-hydroxybenzoat | METHYLPARABEN | 0,15 | 0,37 |
| Titriplex® III | DISODIUM EDTA | 0,05 | 0,13 |
| Water, demineralized | AQUA (WATER) | ad 100 | ad 250 |
| **C** | | | |
| Natronlauge, 10 %ig 10 %ig | AQUA (WATER), SODIUM HYDROXIDE | 0,20 | 0,50 |
| | | **ad100,00** | **ad250,00** |

**Herstellung**

Phase B: Keltrol in Wasser dispergieren. Die restlichen Bestandteile zugeben und mischen.

Phase A und B getrennt auf 80°C erhitzen.

Phase B in Phase A einemulgieren. Homogenesieren.

Unter Rühren abkühlen lassen und unter 35°C pH mit Phase C auf ca. 6,0 einstellen.

In vivo SPF:21,3; UVA-PF: 9,9

Beispiel 5:

Bestimmung des in vitro SPF.

In vitro SPF Bestimmung von kosmetischen Zubereitungen:

Das grundsätzliche Messprinzip welches der Messung zugrunde liegt ist die Bestimmung der UV-Transmission durch eine Zubereitung die Substanzen zum Schutz vor UV-Licht enthält. Hierbei wird die Zubereitung auf ein geeignetes Substrat in definierter Schichtdicke aufgetragen und an einem geeigneten UV-Photometer in nanometer-Schritten die Absorption gemessen. Die Berechnung des in-vitro-Lichtschutzfaktors erfolgt dabei nach folgender Formel:

$$SPF_{vitro} = \frac{\int_{290nm}^{400nm} E(\lambda) * S(\lambda) * \delta(\lambda)}{\int_{290nm}^{400nm} E(\lambda) * S(\lambda) * \delta(\lambda) / MPF(\lambda)} = \frac{\int_{290nm}^{400nm} E(\lambda) * S(\lambda) * \delta(\lambda)}{\int_{290nm}^{400nm} E(\lambda) * S(\lambda) * \delta(\lambda) / 10^{-A(\lambda)}}$$

| | |
|---|---|
| E ($\lambda$) | = Bestrahlungsstärke bei Wellenlänge $\lambda$ des Referenz Sonnenlicht Spektrums |
| S ($\lambda$) | = Erythemale Effektivität bei Wellenlänge $\lambda$ |
| MPF ($\lambda$) | = Monochromatischer Schutzfaktor |
| A ($\lambda$) | = Absorption |

Substrat: PMMA Plexiglasplättchen, Typ XT220070 in Grösse 7,5 cm x 2,5 cm (Firma Roehm, Darmstadt), auf einer Seite mittels Sandstrahlen angerauht (90-150 $\mu$m Glassperlen, 30 bar, 30 cm Abstand). Spezifikation: DIN 67502
Auftragsmenge: 1,25 mg/cm$^2$ $\pm$ 5%
Probenvorbereitung: Möglichst viele kleine Tröpfchen der zu bestimmenden Probe werden mit einer geeigneten Pipette oder einem Spatel auf das Substrat aufgebracht und homogen verteilt. Dabei ist das tara des Substrates, die Auftragsmenge in feuchtem Zustand und nach Equilibrieren (20 min bei Raumtemperatur) zu notieren. Die Equilibrierung erfolgt im Dunkeln. Jede Probe wird auf mindestens drei Substraten gemessen.

Die anschliessende Messung der Absorption erfolgt UV-photometrisch (z.B. Cary 300 Bio (Varian Inc. Palo Alto, USA) mit Ulbrichtkugel (Labsphere DRA-CA-301, North Suttin, USA) über einen Messbereich von 290-400 nm in 1 nm Schritten. Die spektrale Bandbreite beträgt 2 nm.

Vor der Messung der Probe ist eine Basislinie aufzuzeichnen, wobei ein Substrat ohne Probe verwendet wird (100% Transmission). Die nachfolgende Messung der Transmission (resp. Absorption) der Proben erfolgt pro Plättchen an vier Messpunkten. Die Auswertung der insgesamt 12 Messpunkten pro Probe erfolgt mittels geeigneter Software (z.B. Excel).

In vitro SPF [PMMA, 0,75 mg/cm$^2$]
Beispiel C = 23,0
Beispiel D = 4,5.

Beispiel 6:

Bestimmung des SPF Wertes in vivo nach COLIPA (International Sun Protection Factor (SPF) Test Method, COLIPA, May 2006)

**UV Quelle:** 300 Watt Xenon Bogenlampen-Sonnen Simulator (Model 601-300 Multiport, Firma Solar Light Co. Inc. Philadelphia, PA, USA)

**Methode**

Vortest zur Bestimmung der MED (Minimale Erythem Dosis)

6 verschiedene UV-Bestrahlungsdosen (Spot 1 cm Durchmesser) werden auf der Rückenpartie der Testpersonen angewendet. 16 bis 24 Stunden nach der Bestrahlung wird jeweils die MED auf der nicht geschützten Haut der Testperson visuell bestimmt.

Haupttest

Der Haupttest wird auf jeweils 35 $cm^2$ großen Flächen der Rückenpartie durchgeführt.

Produkt Applikation und Standard

Auf jeder Fläche werden 70 mg Produkt aufgetragen, um eine Produktmenge von 2 $mg/cm^2$ (+/- 2,5%) zu erhalten, wobei das Produkt mit einem Handschuh verteilt wird.

Wartezeit

Nach Beendigung der Auftragung des Produktes wird eine Wartezeit von 15 Minuten eingehalten, bevor mit der Bestrahlung begonnen wird.
Vor der Bestrahlung wird jede Testfläche in 6 Zonen aufgeteilt. Jede Zone wird einer unterschiedlichen Bestrahlungsdosis ausgesetzt. Die Bestrahlungszeiten für die einzelnen Testpersonen werden an Hand der in den Vortests bestimmten individuellen MED-Werten festgelegt.
Die Auswertung wird 16-24 Stunden nach Bestrahlung von ausgebildetem Personal durchgeführt.

**Ergebnisse**

Zur Bestimmung eines SPF-Wertes werden jeweils die an 6 Testpersonen ermittelten Ergebnisse herangezogen. Die SPF-Werte sind bei den sich anschließenden Formulierungen angegeben.

Formulierung 1:

| Phase | [Gew.-%] | Rohstoffe | *INCI* |
|---|---|---|---|
| A | | | |
| | 10,0 | Miglyol 812 | Caprylic/Capric Triglycerides |
| | 1,5 | Abil350 | Dimethicone |
| | 3,0 | Finsolv TN | C12-15 Alkyl Benzoate |
| | 1,0 | Cremophor CO 40 | PEG-40 Hydrogenated Castor Oil |
| B | | | |
| | 12,05 | **UV-Filter-Kapsel** (16,6 % Ethylhexyl Triazone in Spectrasolv DMDA) | **Aqua (water), Ethylhexyl Triazone, Dimethyl Capramide, Silica, PVP, Chlorphenesin** |
| C | 5,0 | Propylenglykol | Propylene Glycol |
| | 5,0 | Ethanol | |
| | 0,5 | Cremophor A 25 | Alcohol Ceteareth-25 |
| | Ad 100 | Wasser | Water |
| D | 2,0 | Sepigel 305 | Polyacrylamide, C13-14 Isoparaffin, Laureth-7 |
| E | 1,0 | Euxyl K300 | Phenoxyethanol und Methylparaben and Butylparaben und Ethylparaben and Propylparaben und Isobutylparaben |

Herstellung:

Die Komponenten der Phase A werden auf 80°C erhitzt.
Die Komponenten der Phase B werden bei Raumtemperatur unter Rühren dispergiert und zu der Phase A gegeben.

Die Komponenten der Phase C werden homogenisiert und zu dem Gemisch der Phasen A und B gegeben und noch mal homogenisiert.

Die Komponenten der Phasen D und E werden zu dem Gemisch der Phasen A+B+C gegeben, nochmals homogenisiert und anschließend auf Raumtemperatur abgekühlt.
Viskosität: 42600 mPas
pH: 6,5
SPF in vivo nach COLIPA 6,0.

Formulierung 2:

| Phase | [Gew.-%] | Rohstoffe | *INCI* |
|---|---|---|---|
| A | | | |
| | 10,0 | Miglyol 812 | Caprylic/Capric Triglycerides |
| | 1,5 | Abil 350 | Dimethicone |
| | 3,0 | Finsolv TN | C12-15 Alkyl Benzoate |
| | 1,0 | Cremophor CO 40 | PEG-40 Hydrogenated Castor Oil |
| | 2,0 | Uvinul® T150 | Ethylhexyl Triazone |
| B | ad 100 | Wasser | Water |
| | | | |
| | 5,0 | Propylenglykol | Propylene Glycol |
| C | 5,0 | Ethanol | Alcohol |
| | 0,5 | Cremophor A 25 | Ceteareth-25 |
| D | 2,0 | Sepigel 305 | Polyacrylamide, C13-14 Isoparaffin, Laureth-7 |
| E | 1,0 | Euxyl K300 | Phenoxyethanol und Methylparaben und Butylparaben und Ethylparaben und Propylparaben und Isobutylparaben |

Herstellung:

Die Komponenten der Phase A werden auf 80°C erhitzt.

Die Komponenten der Phase B werden bei Raumtemperatur unter Rühren dispergiert und zu der Phase A gegeben.

Die Komponenten der Phase C werden homogenisiert und zu dem Gemisch der Phasen A und B gegeben und noch mal homogenisiert.

Die Komponenten der Phasen D und E werden zu dem Gemisch der Phasen A+B+C gegeben, nochmals homogenisiert und anschließend auf Raumtemperatur abgekühlt.
Viskosität: 3450 mPas
pH-Wert: 6,5
SPF in vivo COLIPA 6,7

Formulierung 3:

| Gew.-% | Rohstoffe | INCI |
|---|---|---|
| Phase A | | |
| 1,0 | Cremophor A 6 | Ceteareth-6, Stearyl Alcohol |
| 1,0 | Cremophor A 25 | Ceteareth-25 |

(fortgesetzt)

| Gew.-% | Rohstoffe | INCI |
|---|---|---|
| Phase A | | |
| 12,0 | Tegin G | Glycol Stearate SE |
| 10,0 | Myglyol 812 | Caprylic/Capric Triglyceride |
| 10,0 | Witconol APM | PPG-3 Myristyl Ether |
| 1,0 | Cetiol SB 45 | Butyrosperum Parkii (Shea Butter) |
| 2,0 | Uvinul T150 | Ethylhexyl Triazone |
| Phase B | | |
| 3,0 | Glycerin 87% | Glycerin |
| 1,5 | Triethanolamin Care | Triethanolamine |
| 3,0 | Uvinul MS 40 | Benzophenone-4 |
| Add.100 | Wasser dem. | Aqua dem. |
| | | |
| Phase C | | |
| 0,5 | Euxyl K300 | Phenoxyethanol, Methylparaben, Butylparaben, Ethylparaben, Propylparaben, Isobutylparaben |

Herstellung:

Die Komponenten der Phasen A und B werden jeweils getrennt voneinander auf 80°C erhitzt, anschließend zusammen gegeben und kurzzeitig homogenisiert.
Das Gemisch wird auf 40°C abgekühlt und nochmals homogenisiert.
Die Komponente der Phase C wird zu dem Gemisch der Phasen A und B gegeben und alles zusammen homogenisiert.
Viskosität (Brookfield SP6): 3350 mPas
pH-Wert: 7,0
SPF in vivo COLIPA 6,6

Formulierung 4:

| Gew.-% | Rohstoffe | INCI |
|---|---|---|
| Phase A | | |
| 1,0 | Cremophor A 6 | Ceteareth-6, Stearyl Alcohol |
| 1,0 | Cremophor A 25 | Ceteareth-25 |
| 12,0 | Teqin G | Glycol Stearate SE |
| 10,0 | Miglyol 812 | Caprylic/Capric Triglyceride |
| 10,0 | Witconol APM | PPG-3 Myristyl Ether |
| 1,0 | Cetiol SB 45 | Butyrosperum Parkii ( Shea Butter) |
| | | |
| Phase B | | |
| 3,0 | Glycerin 87% | Glycerin |
| 1,5 | Triethanolamin Care | Triethanolamine |

(fortgesetzt)

| | | |
|---|---|---|
| Phase B | | |
| 3,0 | Uvinul MS 40 | Benzophenone-4 |
| Add.100 | Wasser dem. | Aqua dem. |
| Phase C | | |
| 2,0 | **UV-Filter-Kapsel** (16,6 % Ethylhexyl Triazone in Spectrasolv DMDA) entspricht 2 % UvinulT 150 | **Aqua (water), Ethylhexyl Triazone, Dimethyl Capramide, Silica, PVP, Chlorphenesin** |
| Phase D | | |
| 0,5 | Euxyl K300 | Phenoxyethanol, Methylparaben, Butylparaben, Ethylparaben, Propylparaben, Isobutylparaben |

Herstellung:

Die Komponenten der Phasen A und B werden jeweils getrennt voneinander auf 80°C erhitzt, anschließend zusammen gegeben und kurzzeitig homogenisiert.
Die Komponenten der Phase C werden zu dem Gemisch der Phasen A und B gegeben und alles zusammen homogenisiert.
Das Gemisch wird auf 40°C abgekühlt und nochmals homogenisiert.
Die Komponenten der Phase D werden zu dem Gemisch der Phasen A+B+C gegeben und alles zusammen homogenisiert.
Viskosität (Brookfield SP6): 7450 mPas
pH-Wert: 7,3
SPF in vivo COLIPA 6,4.

Formulierung 5: O/W Creme

| % | Inhaltsstoff | Charge (400 g) INCI |
|---|---|---|
| Phase A | | |
| 1,8 | Stearinsäure | Stearic Acid |
| 1,8 | Natriumhydroxid 97% | Sodium Hydroxide |
| 10,0 | Wasser dem. | ***Aqua dem.*** |
| Phase B | | |
| 1,0 | Uvinul MC80 | Ethylhexyl Methoxycinnamate |
| 1,0 | Cetiol OE | Dicaprylyl Ether |
| 1,0 | Finsolv TN | ***C12-15 Alkyl Benzoate*** |
| 1,0 | Cetiol SN | Cetearyl Isononanoate |
| 1,0 | Abil K 4 | Cyclomethicone |
| 0,5 | Lanette O | Cetearyl Alcohol |
| 1,0 | Uvinul T150 | Ethylhexyl Triazone |
| 3,5 | Glycerinmonostearat | Glyceryl Stearate |
| 1,0 | Softisan 100 | Hydrogenated Coco-Glycerides |
| 2,5 | Uvinul A Plus Granular | Diethylamino Hydroxybenzoyl Hexyl Benzoate |

(fortgesetzt)

| | | |
|---|---|---|
| Phase C | | |
| 1,0 | **UV-Filter-Kapsel** (16,6 % Ethylhexyl Triazone in Spectrasolv DMDA) | **Aqua (water), Ethylhexyl Triazone, Dimethyl Capramide, Silica, PVP, Chlorphenesin** |
| 5,0 | 1,2-Propylenglycol Care | ***Propylene Glycol*** |
| 3,0 | Glycerin 87% | ***Glycerin*** |
| 1,0 | Carbopol 934 | ***Carbomer*** |
| 0,3 | Dinatrium EDTA | ***Disodium EDTA*** |
| Add.100 | Wasser dem. | ***Aqua dem.*** |
| Phase D | | |
| 1,9 | Zitronensäure | ***Citric Acid*** |
| Phase E | | |
| 0,5 | Vitamin E-Acetat | Tocopherol Acetate |
| 4,0 | Ethanol 96% | Alcohol |
| 1,0 | Euxyl K 300 | Phenoxyethanol and Methylparaben and Butylparaben and Ethylparaben and Propylparaben and Isobutylparaben |
| | | |

Herstellung:

Verseifung der Phase A 1 h bei 80°C.
Schmelzen und mischen der Phase B bei 80°C.
Vermischen der Komponenten der Phase C und erhitzen auf 80°C (ohne Kapseln).
Zumischen der erfindungsgemäßen Kapseln zu Phase C und homogenisieren.
Zumischen der Phase A+B zu C und homogenisieren.
Zugeben der Phase D.
Unter Rühren abkühlen auf Raumtemperatur und Zugabe der Phase E mit anschließender Homogenisierung.

SPF in vivo COLIPA 12
(UV)APF 4

Formulierung 6: O/W Creme

| % | Inhaltstoff | Charge (400 g) INCI |
|---|---|---|
| Phase A | | |
| 1,8 | Stearinsäure | Stearic Acid |
| 1,8 | Natriumhydroxid 97% | Sodium Hydroxide |
| 10,0 | Wasser dem. | ***Aqua dem.*** |
| Phase B | | |
| 1,0 | Uvinul MC80 | Ethylhexyl Methoxycinnamate |
| 1,0 | Cetiol OE | Dicaprylyl Ether |

(fortgesetzt)

| | | |
|---|---|---|
| Phase B | | |
| 1,0 | Finsolv TN | ***C12-15 Alkyl Benzoate*** |
| 1,0 | Cetiol SN | Cetearyl Isononanoate |
| 1,0 | Abil K 4 | Cyclomethicone |
| 0,5 | lanette O | Cetearyl Alcohol |
| 1,0 | Uvinul T150 | Ethylhexyl Triazone |
| 3,5 | Glycerinmonostearat | Glyceryl Stearate |
| 1,0 | Softisan 100 | Hydrogenated Coco-Glycerides |
| 2,5 | Uvinul A Plus Granular | Diethylamino Hydroxybenzoyl Hexyl Benzoate |
| Phase C | | |
| 1,0 | Eusolex 232 | Phenylbenzimidazole Sulfonic Acid |
| 1,0 | Natriumnydroxid 97% | Sodium Hydroxide |
| 5,0 | 1,2-Propylenglycol Care | ***Propylene Glycol*** |
| 3,0 | Glycerin 87% | ***Glycerin*** |
| 1,0 | Carbopol 934 | ***Carbomer*** |
| 0,3 | Dinatrium EDTA | ***Disodium EDTA*** |
| Add.100 | Wasser dem. | ***Aqua dem.*** |
| Phase D | | |
| 2,6 | Zitronensäure | ***Citric Acid*** |
| Phase E | | |
| 0,5 | Vitamin E-Acetat | Tocopheryl Acetate |
| 4,0 | Ethanol 96% | Alcohol |
| 1,0 | Euxyl K 300 | Phenoxyethanol and Methylparaben and Butylparaben and Ethylparaben and Propylparaben and Isobutylparaben |

Herstellung:

Verseifung der Phase A 1 h bei 80°C.
Schmelzen und mischen der Phase B bei 80°C.
Vermischen der Komponenten der Phase C und erhitzen auf 80°C. Zumischen der Phase A+B zu C und homogenisieren.
Zugeben der Phase D.
Unter Rühren abkühlen auf Raumtemperatur und Zugabe der Phase E mit anschließender Homogenisierung.
SPF in vivo COLIPA 8
(UV)APF 3.8

Formulierung 7: O/W-Emulsion

| % | Inhaltstoff | INCI |
|---|---|---|
| Phase A | | |
| 1,0 | Cremophor A 6 | Ceteareth-6, Stearyl Alcohol |
| 1,0 | Cremophor A 25 | Ceteareth-25 |

(fortgesetzt)

| % | Inhaltstoff | INCI |
|---|---|---|
| Phase A | | |
| 12,0 | Teqin | Glyceryl Stearate SE |
| 10,0 | Miglyol 812 | Caprylic/Capric Triglyceride |
| 10,0 | Witconol APM | PPG-3 Myristyl Ether |
| 1,0 | Cetiol SB 45 | *Butrosperum Parkii (Shea Butter)* |
| 2,0 | Uvinul T150 | Ethylhexyl Triazone |
| Phase B | | |
| 3,0 | Glycerin 87% | Glycerin |
| 1,5 | Triethanolamin Care | Triethanolamine |
| 3,0 | ***Uvinul MS 40*** | Benzophenone-4 |
| Add.10 0 | Wasser dem. | Aqua dem. |
| | | |
| Phase C | | |
| 0,5 | Euxyl K300 | Phenoxyethanol, Methylparaben, Butylparaben, Ethylparaben, Propylparaben, Isobutylparaben |

Herstellung:

Erhitzen der Phasen A und B separat auf 80°C.
Unter Rühren Phase B in Phase A geben und kurz homogenisieren. Zugabe von Phase C in die vereinigeten Phasen A+B und homogenisieren. Abkühlen auf 40°C und homogenisieren.
Zugabe von Phase C und homogenisieren.
Viskosität (Brookfield SP6): 3350 mPas
pH-value: 7,0
SPF in vivo COLIPA 6

Formulierung 8: O/W-Emulsion

| % | Inhaltstoff | INCI |
|---|---|---|
| Phase A | | |
| 1,0 | Cremophor A 6 | Ceteareth-6, Stearyl Alcohol |
| 1,0 | Cremophor A 25 | Ceteareth-25 |
| 12,0 | Tegin G | Glycol Stearate SE |
| 10,0 | Miglyol 812 | Caprylic/Capric Triglyceride |
| 10,0 | Witconol APM | PPG-3 Myristyl Ether |
| 1,0 | Cetiol SB 45 | Butyrosperum Parkii (Shea |
| | | |
| Phase B | | |
| 3,0 | Glycerin 87% | Glycerin |
| 1,5 | Triethanolamin Care | Triethanolamine |

(fortgesetzt)

| | | |
|---|---|---|
| Phase B | | |
| 3,0 | ***vinul MS 40*** | Benzophenone-4 |
| Add.100 | Water dem. | Aqua dem. |
| 2,0 | UV-Filter-Kapsel (16,6 % Ethylhexyl Triazone in Spectrasolv DMDA) | ***Aqua (water), Ethylhexyl Triazone, Dimethyl Capramide, Silica, PVP, Chlorphenesin*** |
| Phase C | | |
| 0,5 | Euxyl K300 | Phenoxyethanol, Methylparaben, Butylparaben, Ethylparaben, Propylparaben, Isobutylparaben |

Herstellung:

Phase A und Phase B getrennt voneinander jeweils auf 80°C erhitzen. Zugabe der Phase B unter Rühren in Phase A und kurzzeitiges homogenisieren. Unter Rühren auf 40°C abkühlen und Phase C zufügen und homogenisieren.
Viskosität (Brookfield SP6): 7450 mPas
pH-value: 7,3
SPF in vivo COLIPA 6

Formulierung 9:

| % | Inhaltstoff | INCI |
|---|---|---|
| Phase A | | |
| 10,0 | Miglyol 812 | Caprylic/Capric Triglycerides |
| 1,5 | Abil350 | Dimethicone |
| 3,0 | Finsolv TN | C12.15 Alkyl Benzoate |
| 1,0 | Cremophor CO 40 | PEG-40 Hydrogenated Castor Oil |
| Phase B | | |
| ad 100 | Wasser | Water |
| 2,0 | UV-Filter-Kapsel (16.6% Ethylhexyl Triazone I Spectrasolv DMDA) | **Aqua (water), Ethylhexyl Triazone, Dimethyl Capramide, Silica, PVP, Chlorphenesin** |
| 5,0 | Propylenglykol | Propylene Glycol |
| Phase C | | |
| 5,0 | Ethanol | Alcohol |
| 0,5 | Cremophor A 25 | Ceteareth-25 |
| Phase D | | |
| 2,0 | Sepigel 305 | Polyacrylamide, C13-14 Isoparaffin, Laureth-7 |
| Phase E | | |
| 1,0 | Euxyl K300 | Phenoxyethanol and Methylparaben and Butylparaben and Ethylparaben and Propylparaben and Isobutylparaben |

Herstellung:

Erhitzen der Phase A auf 80°C.
Dispergieren der Phase B bei Raumtemperatur unter Rühren. Homogenisieren der Phase B in Phase A,
**[0261]** Zugabe der Phase C zu Phase A+B und homogenisieren. Nach Zugabe der Phase D und E wieder homogenisieren.
SPF in vitro 6.

Formulierung 10:

| % | Inhaltstoff | **INCI** |
|---|---|---|
| Phase A | | |
| 10,0 | Miglyol 812 | Caprylic/Capric Triglycerides |
| 1,5 | Abil350 | Dimethicone |
| 3,0 | Finsolv TN | C12.15 Alkyl Benzoate |
| 1,0 | Cremophor CO 40 | PEG-40 Hydrogenated Castor Oil |
| 2,0 | Uvinul T150 | Ethylhexyl Triazone |
| Phase B | | |
| ad 100 | Wasser | Water |
| | | |
| 5,0 | Propylenglykol | Propylene Glycol |
| Phase C | | |
| 5,0 | Ethanol | Alcohol |
| 0,5 | Cremophor A 25 | Ceteareth-25 |
| Phase D | | |
| 2,0 | Sepigel 305 | Polyacrylamide, C13-14 Isoparaffin, Laureth-7 |
| Phase E | | |
| 1,0 | Euxyl K300 | Phenoxyethanol and Methylparaben and Butylparaben and Ethylparaben and Propylparaben and Isobutylparaben |

Herstellung:

Erhitzen der Phase A auf 80°C.
Dispergieren der Phase B bei Raumtemperatur unter Rühren. Homogenisieren der Phase B in Phase A,
**[0266]** Zugabe der Phase C zu Phase A+B und homogenisieren.
Nach Zugabe der Phase D und E wieder homogenisieren.
SPF in vitro 6

Formulierung 11:

| % | Inhaltstoff | Charge (400 g) INCI |
|---|---|---|
| Phase A | | |
| 3,0 | Uvinul T 150 | Ethylhexyl Triazone |
| 10,0 | Uvinul A-Plus Granular | Diethylamino Hydroxybenzoyl Hexyl Benzoate |
| 3,0 | Tinosorb S | Bis-Ethylhexyloxyphenol Methoxyphenyl Triazine |
| 1,0 | Cutina GMS | ***Glyceryl Stearate*** |
| 2,0 | Axol C 62 | Glyceryl Stearate Citrate |
| 7,0 | Finsolv TN | C12-15 Alkyl Benzoate |
| 7,0 | Cetiol B | Dibutyl Adipate |
| 4,0 | Cosmacol ETI | Di-C12-13 Alkyl Tartrate |
| 1,0 | Lanette O | Cetearyl Alcohol |
| 2,0 | Unimer U-6 | Triacontanyl PVP |
| 3,0 | Luvitol Lite | Hydrogenated Polyisobutene |
| Phase B | | |
| 6,0 | T-Lite SF-S | Titanium Dioxide and Hydrated Silica and Dimethicone/Methicone Copolymer and Aluminum Hydroxide |
| Phase C | | |
| 2,0 | Glycerin 86% | ***Glycerin*** |
| 2,0 | Panthenol 50 P | ***Panthenol*** |
| 0,2 | Edeta BD | ***Disodium EDTA*** |
| ad 100 | Wasser, demin. | Water |
| 0,15 | Keltrol [E] | Xanthan Gum |
| 0,15 | Polysurf 67 CS | Cetyl Hydroxyethylcellulose |
| 4,8 | Tris Amino Ultra PC(20% in wasser) | Tromethamine |
| 2,0 | Eusolex 232 | Phenylbenzimidazole Sulfonic Acid |
| Phase D | | |
| 0,5 | Vitamin E-Acetat | Tocopheryl Acetate |
| 1,0 | Euxyl K 300 | Phenoxyethanol and Methylparaben and Butylparaben and Ethylparaben and Propylparaben and Isobutylparaben |
| | | |

Herstellung:

Erhitzen der Phase A auf 80°C.

Zugabe der Phase B zur geschmolzenen Phase A und homogenisieren. Homogenisieren der Phase C bis zur Klarheit.

Zugabe der Phase C zu den Phasen A+B und homogenisieren.

Unter Rühren abkühlen auf 40°C.

Zugabe der Phase D, kurzzeitiges homogenisieren und abkühlen auf Raumtemperatur unter Rühren.

SPF in vivo COLIPA 30

Formulierung 12:

| % | Inhaltstoff | INCI |
|---|---|---|
| Phase A | | |
| 3,0 | Uvinul T 150 | Ethylhexyl Triazone |
| 10,0 | Uvinul A-Plus Granular | Diethylamino Hydroxybenzoyl Hexyl Benzoate |
| 3,0 | Tinosorb S | Bis-Ethylhexyloxyphenol Methoxyphenyl Triazine |
| 1,0 | Cutina GMS | ***Glyceryl Stearate*** |
| 2,0 | Axol C 62 | Glyceryl Stearate Citrate |
| 7,0 | Finsolv TN | C12-15 Alkyl Benzoate |
| 7,0 | Cetiol B | Dibutyl Adipate |
| 4,0 | Cosmacol ETI | Di-C12-13 Alkyl Tartrate |
| 1,0 | Lanette O | Cetearyl Alcohol |
| 2,0 | Unimer U-6 | Triacontanyl PVP |
| 3,0 | Luvitol Lite | Hydrogenated Polyisobutene |
| | | |
| Phase B | | |
| 6,0 | T-Lite SF-S | Titanium Dioxide and Hydrated Silica and Dimethicone/Methicone Copolymer and Aluminum Hydroxide |
| Phase C | | |
| 2,0 | Glycerin 86% | ***Glycerin*** |
| 2,0 | Panthenol 50 P | ***Panthenol*** |
| 0,2 | Edeta BD | ***Disodium EDTA*** |
| ad 100 | Wasser, demin. | Water |
| 0,15 | Keltrol [E] | Xanthan Gum |
| 0,15 | Polysurf 67 CS | Cetyl Hydroxyethylcellulose |
| 4,8 | Tris Amino Ultra PC(20% in water) | Tromethamine |
| 12,05 | UV-Filter-Kapsel (16.6% Ethylhexyl Triazone in Spectrasolv DMDA) | **Aqua (water), Ethylhexyl Triazone, Dimethyl Capramide, Silica, PVP, Chlorphenesin** |
| Phase D | | |
| 0,5 | Vitamin E-Acetat | Tocopheryl Acetate |
| 1,0 | Euxyl K 300 | Phenoxyethanol and Methylparaben and Butylparaben and Ethylparaben and Propylparaben and Isobutylparaben |
| | | |

Herstellung:

Erhitzen der Phase A auf 80°C.

Zugabe der Phase B zur geschmolzenen Phase A and homogenisieren.

Homogenisieren der Phase C bis zur Klarheit. Zugabe der Phase C zu den Phasen A+B und homogenisieren.

Abkühlen auf 40°C unter Rühren.

Zugabe der Phase D, kurzzeitiges homogenisieren und unter Rühren Abkühlen auf Raumtemperatur.

SPF in vivo COLIPA 33
(UV)APF in vitro 11.

**Patentansprüche**

1. UV-Filter-Kapsel, umfassend
eine polymere Hülle und

    a) mindestens einen organischen UV-Filter mit einer Löslichkeit in Dicaprylyl Carbonate von weniger als 40% bei 20°C bis 25°C und einer Versushdauer von 24 Stunden; und
    b) ein Emollient, welches in der Lage ist, bei Raumtemperatur mehr als 40 Gew.% des organischen UV-Filters a) zu lösen, **dadurch gekennzeichnet, dass** das unter b) genannte Emollient einer Verbindung der Formel I entspricht

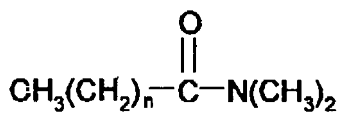

I

    wobei n einer ganzen Zahl von 2 bis 12 entspricht.

2. UV-Filter-Kapsel nach Anspruch 1, **dadurch gekennzeichnet, dass** es sich bei dem unter a) genannten UV-Filter um ein Triazinderivat, Diarylbutadienderivat, Hydroxybenzophenonderivat und/oder Methylen bis-benzotriazolyltetramethylbutylphenolderivat handelt.

3. UV-Filter-Kapsel nach einem Anspruch 1 oder 2, wobei der unter a) genannte organische UV-Filter ein Triazinderivat ist, ausgewählt aus der Gruppe enthaltend 2,4,6-Tris-[anilino-(p-carbo-2'-ethyl-1'-hexyloxy)]-1,3,5-triazin, Dioctylbutamidotriazon, Bis-Ethylhexyloxyphenol-methoxyphenyltriazin, 2,4,6-tris(diethyl-4'-amino-benzalmalonate)-s-triazin, 2,4,6-tris(dimethyl-4'-amino-benzalmalonate-s-triazin, 2,4,6-tris(diisopropyl-4-aminobenzal-malonate)-s-triazin, 2,4,6-tris[3'-benzotriazol-2-yl)-2'-hydroxy-5'-methyl)phenyl-amino]-s-triazin und 2,4,6-tris[3'benzotriazol-2-yl)-2'-hydroxy-5'-tert-octyl)phenyl-amino]-s-triazin.

4. UV-Filter-Kapsel nach einem oder mehreren der Ansprüche 1 bis 3, wobei der unter a) genannte organische UV-Filter ein Triazinderivat ist, ausgewählt aus 2,4,6-Tris-[anilino-(p-carbo-2'-ethyl-1'-hexyloxy)]-1,3,5-triazin, Dioctylbutamidotriazon oder Bis-Ethylhexyloxyphenol-methoxyphenyltriazin.

5. UV-Filter-Kapsel nach Anspruche 1 oder 2, wobei der unter a) genannte organische UV-Filter ein Diarylbutadienderivat ist und das Diarylbutadien der Formel II entspricht,

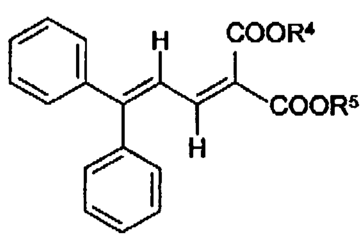

II

,

wobei $R^4$ und $R^5$ unabhängig voneinander Wasserstoff, $C_1$-$C_{20}$-Alkyl, $C_3$-$C_{10}$-Cycloalkyl oder $C_3$-$C_{10}$-Cycloalkenyl bedeuten.

6. UV-Filter-Kapsel nach einem oder mehreren der Ansprüche 1,2 und 5, wobei der unter a) genannte

organische UV-Filter 1,1-Dicarboxy(2',2'-dimethylpropyl)-4-4-diphenylbutadien ist.

**7.** UV-Filter-Kapsel nach Anspruch 1 oder 2, wobei der unter a) genannte organische UV-Filter einem Hydroxybenzophenon der Formel III entspricht,

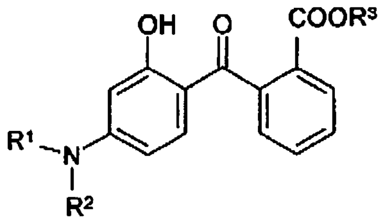

III

,

wobei $R^1$ und $R^2$ unabhängig voneinander H, $C_1$-$C_{20}$-Alkyl, $C_3$-$C_{10}$-Cycloalkyl oder $C_3$-$C_{10}$-Cycloalkenyl bedeuten, wobei die Substituenten $R^1$ und $R^2$ gemeinsam mit dem Stickstoffatom, an das sie gebunden sind, einen 5- oder 6-Ring bilden können und $R^3$ $C_1$-$C_{20}$-Alkyl bedeutet.

**8.** UV-Filter-Kapsel nach einem oder mehreren der Ansprüche 1, 2 und 7, wobei der unter a) genannte organische UV-Filter 2-(4-Diethylamino-2-hydroxybenzoyl)-benzoesäure-hexylester ist.

**9.** UV-Filter-Kapsel nach Anspruch 1 oder 2, wobei der unter a) genannte organische UV-Filter ein Methylen bis-Benzotriazolyl Tetramethylbutylphenolderivat ist.

**10.** UV-Filter-Kapsel nach einem oder mehreren der Ansprüche 1, 2 oder 9, wobei der unter a) genannte organische UV-Filter 2,2'-Methylenbis[6-(2H-benzotriazol-2-yl)-4-(1,1,3,3-tetramethylbutyl)phenol] ist.

**11.** UV-Filter-Kapsel nach einem oder mehreren der Ansprüche 1 bis 10, **dadurch gekennzeichnet, dass** die UV-Filter-Kapsel den unter a) genannten UV-Filter und das unter b) genannte Emollient im Gewichtsprozentverhältnis 10:90 bis 90:10, vorzugsweise im Gewichtsprozentverhältnis 30:70 bis 70:30 enthält.

**12.** Dispersion enthaltend UV-Filter-Kapseln nach einem oder mehreren der Ansprüche 1 bis 11.

**13.** Dispersion nach Anspruch 12, wobei die Dispersion wässrig ist.

**14.** Dispersion nach Anspruch 12 oder 13, wobei der Anteil an UV-Filter-Kapseln bei 5 bis 80 Gew.-% liegt, bezogen auf die Gesamtmenge der Dispersion.

**15.** Zubereitung enthaltend mindestens einen organischen UV-Filter mit einer Löslichkeit in Dicaprylyl Carbonate von weniger als 40% bei 20°C bis 25°C und einer Versuchsdauer von 24 Stunden; und mindestens einen geeigneten Träger, **dadurch gekennzeichnet, dass** mindestens ein Teil des dieses organischen UV-Filters verkapselt in Form von UV-Filter-Kapseln nach mindestens einem der Ansprüche 1 bis 12 vorliegt.

**16.** Verwendung von UV-Filter-Kapseln nach einem oder mehreren der Ansprüche 1 bis 11 oder einer Dispersion nach einem oder mehreren der Ansprüche 12 bis 14 zur Herstellung einer Zubereitung.

**17.** Verfahren zur Herstellung einer Zubereitung, **dadurch gekennzeichnet, dass** UV-Filter-Kapseln nach einem oder mehreren der Ansprüche 1 bis 11 oder eine Dispersion nach einem oder mehreren der Ansprüche 12 bis 14 mit weiteren Inhaltsstoffen vermischt werden oder wird.

**18.** Verfahren zur Herstellung von UV-Filter-Kapseln nach einem oder mehreren der Ansprüche 1 bis 11, **dadurch gekennzeichnet, dass**
in Schritt a) eine Öl-in-Wasser-Emulsion aus einer Mischung, enthaltend einen Sol-Gel-Vorläufer zur Herstellung der polymeren Hülle, mindestens einen UV-Filter mit einer Löslichkeit in Dicaprylyl Carbonate von weniger als 40% bei 20°C bis 25°C und einer Versuchsdauer von 24 Stunden und ein Emollient, welches in der Lage ist, bei Raumtemperatur mehr als 40 Gew.-%
dieses organischen UV-Filters zu lösen und der Formel I entspricht, in einer wässrigen Lösung hergestellt wird,

in Schritt b) die in Schritt a) hergestellte Emulsion zu einer wässrigen Lösung mit einem pH von 2 bis 4 gemischt wird und gegebenenfalls
in Schritt c) Reaktionsprodukte aus dem Sol-Gel-Vorläufer abgetrennt werden und die UV-Filter-Kapseln isoliert werden.

## Claims

1. A UV filter capsule comprising
a polymeric coating and

   a) at least one organic UV filter with a solubility in dicaprylyl carbonate of less than 40% at 20°C to 25°C and an experiment time of 24 hours; and
   b) an emollient which is able to dissolve more than 40% by weight of the organic UV filter a) at room temperature, wherein the emollient specified under b) corresponds to a compound of the formula I

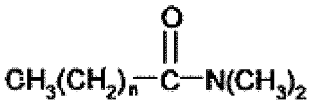

I

where n corresponds to an integer from 2 to 12.

2. The UV filter capsule according to Claim 1, wherein the UV filter specified under a) is a triazine derivative, diarylbutadiene derivative, hydroxybenzophenone derivative and/or methylene bis-benzotriazolyltetramethylbutylphenol derivative.

3. The UV filter capsule according to either Claim 1 or 2, wherein the organic UV filter specified under a) is a triazine derivative selected from the group comprising 2,4,6-tris[anilino (p-carbo-2'-ethyl-1'-hexyloxy)]-1, 3, 5-triazine, dioctylbutamidotriazone, bis-ethylhexyloxyphenol-methoxy-phenyltriazine, 2,4,6-tris(diethyl-4'-amino-benzalmalonate)-s-triazine, 2,4,6-tris(dimethyl-4'-amino-benzalmalonate-s-triazine, 2,4,6-tris(diisopropyl-4-aminobenzalmalonate)-s-triazine, 2,4,6-tris[3'-benzotriazol-2-yl)-2'-hydroxy-5'-methyl) phenyl-amino]-s-triazine and 2,4,6-tris[3'benzotriazol-2-yl)-2'-hydroxy-5'-tert-octyl)phenyl-amino]-s-triazine.

4. The UV filter capsule according to one or more of Claims 1 to 3, wherein the organic UV filter specified under a) is a triazine derivative selected from 2,4,6-tris[anilino(p-carbo-2'-ethyl-1'-hexyloxy)]-1,3,5-triazine, dioctylbutamidotriazone or bis-ethylhexyloxyphenol-methoxy-phenyltriazine.

5. The UV filter capsule according to Claim 1 or 2, wherein the organic UV filter specified under a) is a diarylbutadiene derivative and the diarylbutadiene corresponds to the formula II

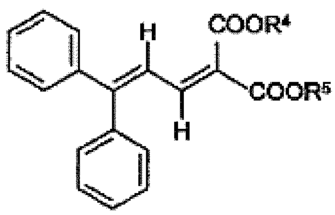

II

,

where $R^4$ and $R^5$, independently of one another, are hydrogen, $C_1$-$C_{20}$-alkyl, $C_3$-$C_{10}$-cycloalkyl or $C_3$-$C_{10}$-cycloalkenyl.

6. The UV filter capsule according to one or more of Claims 1, 2 and 5, wherein the organic UV filter specified under a) is 1, 1-dicarboxy(2',2'-dimethylpropyl)-4-4-diphenylbutadiene.

7. The UV filter capsule according to Claim 1 or 2, wherein the organic UV filter specified under a) corresponds to a hydroxybenzophenone of the formula III,

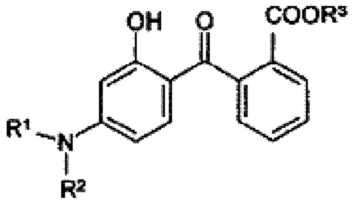

III

,

where $R^1$ and $R^2$, independently of one another, are H, $C_1$-$C_{20}$-alkyl, $C_3$-$C_{10}$-cycloalkyl or $C_3$-$C_{10}$-cycloalkenyl, where the substituents $R^1$ and $R^2$, together with the nitrogen atom to which they are bonded, can form a 5-or 6-membered ring and $R^3$ is $C_1$-$C_{20}$-alkyl .

**8.** The UV filter capsule according to one or more of Claims 1, 2 and 7, wherein the organic UV filter specified under a) is 2-(4-diethylamino-2-hydroxybenzoyl) benzoic acid hexyl ester.

**9.** The UV filter capsule according to Claim 1 or 2, wherein the organic UV filter specified under a) is a methylene bis-benzotriazolyl tetramethylbutylphenol derivative.

**10.** The UV filter capsule according to one or more of Claims 1, 2 or 9, wherein the organic UV filter specified under a) is 2,2'-methylenebis[6-(2H-benzotriazol-2-yl)-4-(1,1,3,3-tetramethylbutyl)phenol].

**11.** The UV filter capsule according to one or more of Claims 1 to 10, wherein the UV filter capsule comprises the UV filter specified under a) and the emollient specified under b) in the percent by weight ratio 10:90 to 90:10, preferably in the percent by weight ratio 30:70 to 70:30.

**12.** A dispersion comprising UV filter capsules according to one or more of Claims 1 to 11.

**13.** The dispersion according to Claim 12, wherein the dispersion is aqueous.

**14.** The dispersion according to Claim 12 or 13, wherein the fraction of UV filter capsules is 5 to 80% by weight, based on the total amount of the dispersion.

**15.** The preparation comprising at least one organic UV filter with a solubility in dicaprylyl carbonate of less than 40% at 20°C to 25°C and an experiment time of 24 hours; and at least one suitable carrier, wherein at least some of this organic UV filter is present encapsulated in the form of UV filter capsules according to at least one of Claims 1 to 12.

**16.** The use of UV filter capsules according to one or more of Claims 1 to 11 or of a dispersion according to one or more of Claims 12 to 14 for producing a preparation.

**17.** A process for producing a preparation, wherein UV filter capsules according to one or more of Claims 1 to 11 or a dispersion according to one or more of Claims 12 to 14 is or are mixed with further ingredients.

**18.** A process for producing UV filter capsules according to one or more of Claims 1 to 11, wherein
in step a) an oil-in-water emulsion is prepared from a mixture comprising a sol gel precursor for producing the polymeric coating, at least one UV filter with a solubility in dicaprylyl carbonate of less than 40% at 20°C to 25°C and an experiment time of 24 hours and an emollient which is able to dissolve more than 40% by weight of this organic UV filter at room temperature and corresponds to formula I, in an aqueous solution,
in step b) the emulsion prepared in step a) is mixed to give an aqueous solution with a pH of 2 to 4 and optionally
in step c) reaction products are separated off from the sol gel precursor and the UV filter capsules are isolated.

**Revendications**

**1.** Capsule filtrant les UV, comprenant une enveloppe polymère et

a) au moins un filtre organique des UV présentant une solubilité dans le carbonate de dicaprylyle inférieure à 40% à 20°C jusqu'à 25°C et pendant une durée d'essai de 24 heures ; et

b) un émollient qui est en mesure de dissoudre, à température ambiante, plus de 40% en poids du filtre organique des UV a),
**caractérisée en ce que** l'émollient mentionné sous b)
correspond à un composé de formule I

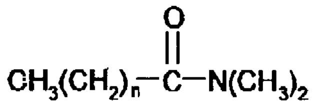

I

n valant un nombre entier de 2 à 12.

2. Capsule filtrant les UV selon la revendication 1, **caractérisée en ce qu'**il s'agit, pour le filtre des UV mentionné au point a), d'un dérivé de triazine, d'un dérivé de diarylbutadiène, d'un dérivé d'hydroxybenzophénone et/ou d'un dérivé de méthylène bis-benzotriazolyl-tétraméthylbutylphénol.

3. Capsule filtrant les UV selon la revendication 1 ou 2, le filtre organique des UV mentionné au point a) étant un dérivé triazinique, choisi dans le groupe contenant la 2,4,6-tris-[anilino-(p-carbo-2'-éthyl-1'-hexyloxy)]-1,3,5-triazine, la dioctylbutamidotriazone, la bis-éthylhexyloxyphénol-méthoxyphényltriazine, la 2,4,6-tris(diéthyl-4'-aminobenzalmalonate)-s-triazine, la 2,4,6-tris(diméthyl-4'-aminobenzalmalonate-s-triazine, la 2,4,6-tris(diisopropyl-4-aminobenzalmalonate)-s-triazine, la 2,4,6-tris[3'-benzotriazol-2-yl)-2'-hydroxy-5'-méthyl)phénylamino]-s-triazine et la 2,4,6-tris[3'-benzotriazol-2-yl)-2'-hydroxy-5'-tert-octyl)phénylamino]-s-triazine.

4. Capsule filtrant les UV selon l'une ou plusieurs des revendications 1 à 3, le filtre organique des UV mentionné au point a) étant un dérivé triazinique choisi parmi la 2,4,6-tris-[anilino-(p-carbo-2'-éthyl-1'-hexyloxy)]-1,3,5-triazine, la dioctylbutamidotriazone ou la bis-éthylhexyloxyphénol-méthoxyphényltriazine.

5. Capsule filtrant les UV selon la revendication 1 ou 2, le filtre organique des UV mentionné au point a) étant un dérivé de diarylbutadiène et correspondant au diarylbutadiène de formule II

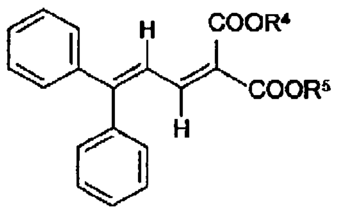

II

,

$R^4$ et $R^5$ signifiant, indépendamment l'un de l'autre, hydrogène, $C_1$-$C_{20}$-alkyle, $C_3$-$C_{10}$-cycloalkyle ou $C_3$-$C_{10}$-cycloalcényle.

6. Capsule filtrant les UV selon l'une ou plusieurs des revendications 1, 2 et 5, le filtre organique des UV mentionné au point a) étant le 1,1-dicarboxy-(2',2'-diméthylpropyl)-4-4-diphénylbutadiène.

7. Capsule filtrant les UV selon la revendication 1 ou 2, le filtre organique des UV mentionné au point

a) étant une hydroxybenzophénone de formule III

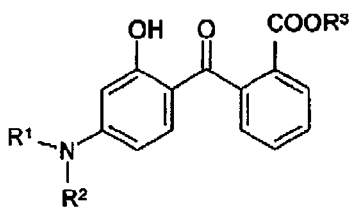

III

,

$R^1$ et $R^2$ signifiant, indépendamment l'un de l'autre, H, $C_1$-$C_{20}$-alkyle, $C_3$-$C_{10}$-cycloalkyle ou $C_3$-$C_{10}$-cycloalcényle, les substituants $R^1$ et $R^2$ pouvant former, avec l'atome d'azote auquel ils sont liés, un cycle de 5 ou 6 chaînons et $R^3$ signifiant un radical $C_1$-$C_{20}$-alkyle.

**8.** Capsule filtrant les UV selon l'une ou plusieurs des revendications 1, 2 et 7, le filtre organique des UV mentionné au point a) étant l'ester hexylique de l'acide 2-(4-diéthylamino-2-hydroxybenzoyl)benzoïque.

**9.** Capsule filtrant les UV selon la revendication 1 ou 2, le filtre organique des UV mentionné au point a) étant un dérivé de méthylène bis-benzotriazolyl-tétraméthylbutylphénol.

**10.** Capsule filtrant les UV selon l'une ou plusieurs des revendications 1, 2 ou 9, le filtre organique des UV mentionné au point a) étant 2,2'-méthylène-bis[6-(2H-benzotriazol-2-yl)-4-(1,1,3,3-tétraméthylbutyl)phénol]

**11.** Capsule filtrant les UV selon l'une ou plusieurs des revendications 1 à 10, **caractérisée en ce que** la capsule filtrant les UV contient les filtres UV mentionnés au point a) et l'émollient mentionné au point b) dans un rapport des pourcentages en poids de 10:90 à 90:10, de préférence dans un rapport de pourcentages en poids de 30:70 à 70:30.

**12.** Dispersion contenant les capsules filtrant les UV selon l'une ou plusieurs des revendications 1 à 11.

**13.** Dispersion selon la revendication 12, la dispersion étant aqueuse.

**14.** Dispersion selon la revendication 12 ou 13, la proportion de capsules filtrant les UV étant de 5 à 80% en poids, par rapport à la quantité totale de la dispersion.

**15.** Composition contenant au moins un filtre organique des UV présentant une solubilité dans le carbonate de dicaprylyle inférieure à 40% à 20°C jusqu'à 25°C et pendant une durée d'essai de 24 heures ; et au moins un support approprié, **caractérisée en ce qu'**au moins une partie de ce filtre organique des UV se trouve sous forme encapsulée dans des capsules filtrant les UV selon au moins l'une quelconque des revendications 1 à 12.

**16.** Utilisation de capsules filtrant les UV selon l'une ou plusieurs des revendications 1 à 11 et d'une dispersion selon l'une ou plusieurs des revendications 12 à 14 pour la préparation d'une composition.

**17.** Procédé pour la préparation d'une composition, **caractérisé en ce qu'**on mélange des capsules filtrant les UV selon l'une ou plusieurs des revendications 1 à 11 ou une dispersion selon l'une ou plusieurs des revendications 12 à 14 avec d'autres constituants.

**18.** Procédé pour la préparation de capsules filtrant les UV selon l'une ou plusieurs des revendications 1 à 11, **caractérisé en ce que** dans l'étape a), on prépare une émulsion huile-dans-eau à partir d'un mélange contenant un précurseur de sol-gel pour la préparation de l'enveloppe polymère, au moins un filtre des UV présentant une solubilité dans le carbonate de dicaprylyle inférieure à 40% à 20°C jusqu'à 25°C et pendant une durée d'essai de 24 heures et un émollient qui est en mesure de dissoudre, à température ambiante, plus de 40% en poids de ce filtre organique des UV et qui correspond à la formule I, dans une solution aqueuse, dans l'étape b), on ajoute l'émulsion préparée dans l'étape a) à une solution aqueuse présentant un pH de 2 à 4 et, le cas échéant, dans l'étape c), on sépare les produits de réaction du précurseur de sol-gel et on isole les capsules filtrant les UV.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 6537529 B
- US 6242099 B1
- WO 0009652 A
- WO 0072806 A
- WO 0071084 A
- WO 0339510 A
- WO 03066209 A
- EP 0796851 A
- EP 0087098 A
- EP 0850935 A
- EP 0967200 A
- EP 916335 A
- DE 11917906 A
- FR 2440933
- DE 10232595
- WO 03007906 A
- DE 10244282 A
- DE 10133202 A
- EP 0671161 A
- DE 4116123 A


### In der Beschreibung aufgeführte Nicht-Patentliteratur


- Handbüchern der Kosmetik. **SCHRADER.** Grundlagen und Rezepturen der Kosmetika. Hüthig Verlag, 1989
- **UMBACH.** Kosmetik: Entwicklung, Herstellung und Anwendung kosmetischer Mittel. Georg Thieme Verlag, 1995
- Römpp Chemie Lexikon. 1993, vol. 9
- **K. LEMANSKA ; H. SZYMUSIAK ; B. TYRAKOWSKA ; R. ZIELINSKI ; I.M.C.M. RIETJENS.** *Current Topics in Biophysics,* 2000, vol. 24 (2), 101-108
- **C.A. RICE-EVANS ; N.J. MILLER ; G. PAGANGA.** *Trends in Plant Science,* 1997, vol. 2 (4), 152-159
- **K. LEMANSKA ; H. SZYMUSIAK ; B. TYRAKOWSKA ; R. ZIELINSKI ; A.E.M.F. SOFFERS ; I.M.C.M. RIETJENS.** *Free Radical Biology&Medicine,* 2001, vol. 31 (7), 869-881
- Rowe Colour Index. Society of Dyers and Colourists, 1971